# EUROPEAN PATENT APPLICATION

(11) **EP 4 485 470 A1**
(43) Date of publication of application: **01.01.2025**
(21) Application number: 24183026.4
(22) Date of filing: 19.06.2024
(51) Int. Cl.: G16H 10/40, G16H 20/60, G16H 30/40

(54) **INFORMATION PROCESSING DEVICE AND PROGRAM**

(30) Priority: 20.06.2023 JP 2023101090
(71) Applicant: Yukashikado Inc., Tokyo 150-0001 (JP)
(72) Inventor: MINOBE, Shinya, Tokyo, 150-0001 (JP)
(74) Representative: TBK

(57) **Abstract**

An information processing device includes an acquisition unit that acquires a concentration of each of a plurality of components included in a body fluid of a living body, and a calculation unit that calculates a difference between the acquired concentration of each component included in the body fluid of the living body and a target concentration of each component included in a body fluid of a target living body determined by attribute data of the living body.

## Description

### Cross Reference to Related Application

This application claims priority to Japanese Patent Application No. 2023-101090, filed June 20, 2023, the contents of which are incorporated herein by reference.

### BACKGROUND OF THE INVENTION

### 1. TECHNICAL FIELD

The technology of the present disclosure relates to an information processing device, a program, a communication terminal, a payment amount calculation device, a point addition device, a product providing device, an information giving device, a shelf, and a movement amount calculation device.

### 2. DESCRIPTION OF THE RELATED ART

Japanese Patent No. 6098166 discloses a store introduction system that includes a user terminal, a store terminal, and a server device and introduces a store (restaurant) that can provide food and drink suitable for health of the user on the basis of a health state of the user.

The user terminal measures nutrients, contents thereof, and calories included in the food and drink taken in by the user. A spectral image of each wavelength from which characteristic amounts with respect to lipids, carbohydrates, proteins, and moisture are obtained is acquired, component analysis of a food to be measured is performed based on the spectral image, a component content of each component is calculated, and calories are calculated. Each element (age, height, weight, or the like) of vital data is input to the user terminal through an input unit of a user terminal device. The user terminal transmits these pieces of data to the server device.

The server device calculates excess and deficient nutrient amounts and excess and deficient calories from an intake amount and calorie of each nutrient of the user and an ideal nutrient amount and an ideal calorie, and extracts a store (restaurant) that can provide food and drink suitable for the health of the user.

### SUMMARY OF THE INVENTION

In order to introduce a store (restaurant) that can provide food and drink suitable for the health of the user, the store introduction system of Japanese Patent No. 6098166 measures nutrients, contents thereof, and calories contained in the food and drink taken in by the user. In this manner, with the nutrients contained in the food and drink taken by the user, a state of a body fluid of the user can be grasped only indirectly.

An object of the technology of the present disclosure is to provide an information processing device, a program, and a communication terminal capable of directly grasping a state of a body fluid of a living body.

Furthermore, an object of the technology of the present disclosure is to provide a payment amount calculation device, a point addition device, a product providing device, a communication terminal, an information giving device, a shelf, and a movement amount calculation device capable of providing a benefit corresponding to information related to the state of the body fluid of the living body.

In order to achieve the above object, an information processing device according to a first aspect of the technology of the present disclosure includes: an acquisition unit that acquires a concentration of each of a plurality of components included in a body fluid of a living body; and a calculation unit that calculates a difference between the acquired concentration of each component of the body fluid of the living body and a target concentration of each component of a body fluid of a target living body determined by attribute data of the living body.

The technology of the present disclosure can directly grasp the body fluid of the living body to grasp a state of the living body.

Furthermore, the technology of the present disclosure can provide a benefit corresponding to the state of a living body.

### BRIEF DESCRIPTION OF THE DRAWINGS

FIG. 1 is a view illustrating a state in which a reagent carrier is input into a container for storing urine;
FIG. 2 is a block diagram of a solution providing system;
FIG. 3 is a block diagram of a smartphone;
FIG. 4A is a block diagram of a server;
FIG. 4B is a diagram illustrating store information;
FIG. 4C is a diagram illustrating history data of a user;
FIG. 5 is a diagram illustrating processing contents of a communication control unit;
FIG. 6 is a diagram illustrating processing contents of an acquisition unit;
FIG. 7 is a diagram illustrating processing contents of a calculation unit and a reading unit;
FIG. 8 is a timing chart illustrating an operation example of a smartphone and a server;
FIG. 9 is a diagram illustrating a screen for displaying each piece of information received from a server of a display device of a smartphone;
FIG. 10 is a view illustrating a screen for displaying a nutrition type of the display device of the smartphone;
FIG. 11 is a block diagram of a convenience store device;
FIG. 12 is a timing chart illustrating an operation example of the smartphone and the convenience store device;
FIG. 13 is a block diagram of a first product providing device;
FIG. 14 is a timing chart illustrating an operation example of the smartphone and the first product providing device;
FIG. 15A is a block diagram of a second product providing device;
FIG. 15B is a timing chart illustrating an operation example of the smartphone and the second product providing device;
FIG. 16 is a timing chart illustrating an operation example of a restaurant device and the server;
FIG. 17 is a block diagram of a supermarket device;
FIG. 18 is a timing chart illustrating an operation example of the smartphone and the supermarket device;
FIG. 19 is a diagram illustrating a nutrition type, a name of a product, and current positions of a designated product and a user in a selling area of a supermarket;
FIG. 20 is a timing chart illustrating an operation example of the smartphone and a server of an EC site;
FIG. 21A is a diagram illustrating an order screen of an ingested material of each nutrition type downloaded from the server of the EC site of the smartphone;
FIG. 21B is a diagram illustrating an order screen illustrating a state in which a representative ingested material in the nutrition type designated by the user is provisionally ordered;
FIG. 21C is a timing chart illustrating an operation example of the smartphone and the server in the case of ordering a reagent carrier;
FIG. 22 is a block diagram of an information giving device;
FIG. 23 is a front view illustrating a shelf that are set in a convenience store, are partitioned according to each nutrition type, and store ingested materials recommended to be ingested corresponding to each nutrition type;
FIG. 24 is a flowchart of partition plate movement amount calculation processing for calculating a movement amount of a partition plate partitioning a storage area of the shelf;
FIG. 25 is a view illustrating a state in which a reagent carrier is input into a container for storing urine according to a first modification; and
FIG. 26 is a diagram illustrating processing contents of an acquisition unit according to a first modification.

### DETAILED DESCRIPTION

Hereinafter, embodiments of the technology of the present disclosure will be described with reference to the drawings.

### [Embodiment]

### (Configuration)

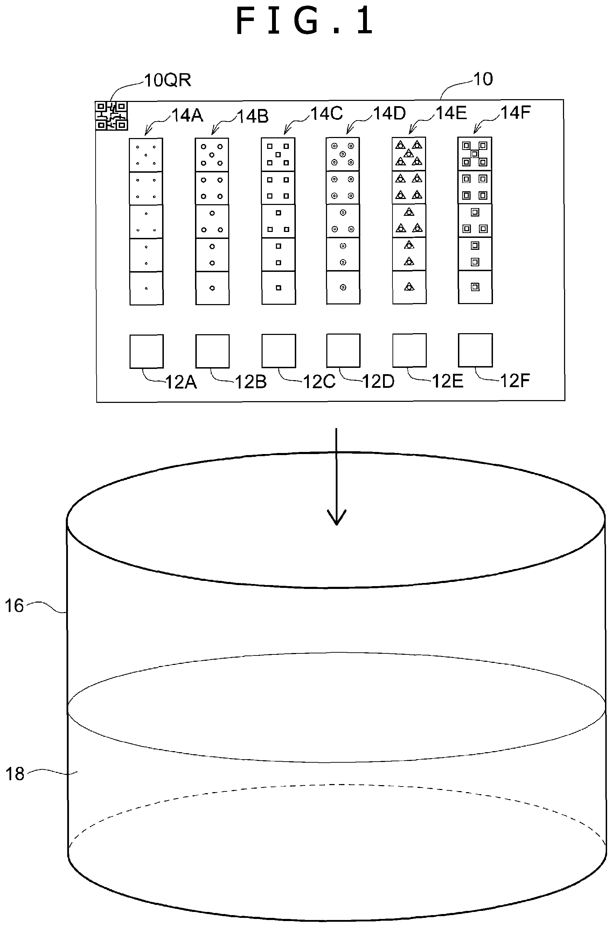

FIG. 1 is a view illustrating a state in which a reagent carrier 10 is input into a container 16 in which urine 18 is contained.

The reagent carrier 10 carries a plurality of reagents 12A to 12F that react with each of a plurality of components of a body fluid of a living body to change a color development state according to the concentration of each component. In the present embodiment, a case where the reagent carrier 10 includes six reagents 12A to 12F that react with each of the six components and change the color development state according to the concentration of each component will be described as an example. The number of components and reagents is not limited to 6, and may be, for example, 2 to 5, 7 to 10, or the like.

The living body is, for example, a human, and the body fluid is, for example, urine. The living body may be an animal other than a human, for example, a cow, a horse, a pig, or the like. The body fluid may be blood, saliva, sweat, or the like other than urine. The color development state includes, for example, a state of color and concentration, but in the present embodiment, the color development state is a state of concentration. Specifically, the reagent 12A reacts with a first component (1) of the urine 18 to change in concentration thereof changes according to the concentration of the first component. The reagent 12B reacts with a second component (2) of the urine 18 to change in concentration according to the concentration of the second component. The reagent 12C reacts with a third component (3) (for example, uric acid) of the urine 18 to change in concentration according to the concentration of the third component. The reagent 12D reacts with a fourth component (4) of the urine 18 to change in concentration according to the concentration of the fourth component. The reagent 12E reacts with a fifth component (5) of the urine 18 to change in concentration according to the concentration of the fifth component. The reagent 12F reacts with the sixth component (6) of the urine 18 to change in concentration according to the concentration of the third component.

The first component (1) to the sixth component (6) are, for example, proteins, coenzyme vitamins, antioxidant vitamins, subtle minerals, macro-minerals, pH.

The reagent carrier 10 carries a color chart. Specifically, the reagent carrier 10 carries a plurality of images 14A of each of a plurality of concentrations of the first component of the urine 18, a plurality of images 14B of each of a plurality of concentrations of the second component of the urine 18, and a plurality of images 14C of each of a plurality of concentrations of the third component of the urine 18. The reagent carrier 10 carries a plurality of images 14D of each of a plurality of concentrations of the fourth component of the urine 18, a plurality of images 14E of each of a plurality of concentrations of the fifth component of the urine 18, and a plurality of images 14F of each of a plurality of concentrations of the sixth component of the urine 18.

For example, the plurality of images 14A are arranged corresponding to the reagent 12A, and are images having lower concentrations in order of proximity to the reagent 12A. In the image closest to the reagent 12A, the concentration of the first component is, for example, 10%, and the concentration increases in order of 20%, 30%, 40%, and 50% as the distance from the reagent 12A increases. For example, it can be understood that the concentration of the first component matching the concentration of the third image in the order of the concentration of the reagent 12A closer to the reagent 12A is 30%. Similarly to the plurality of images 14A, the plurality of images 14B to 14F are also arranged corresponding to the reagents 12B to 12F, and are images having lower concentrations in the order of proximity to the reagents 12B to 12F.

A QR code 10QR is printed on the reagent carrier 10. The QR code 10QR includes information for identifying a shop of the reagent carrier 10.

The reagent carrier 10 is, for example, a support made of paper, plastic, or nonwoven fabric.

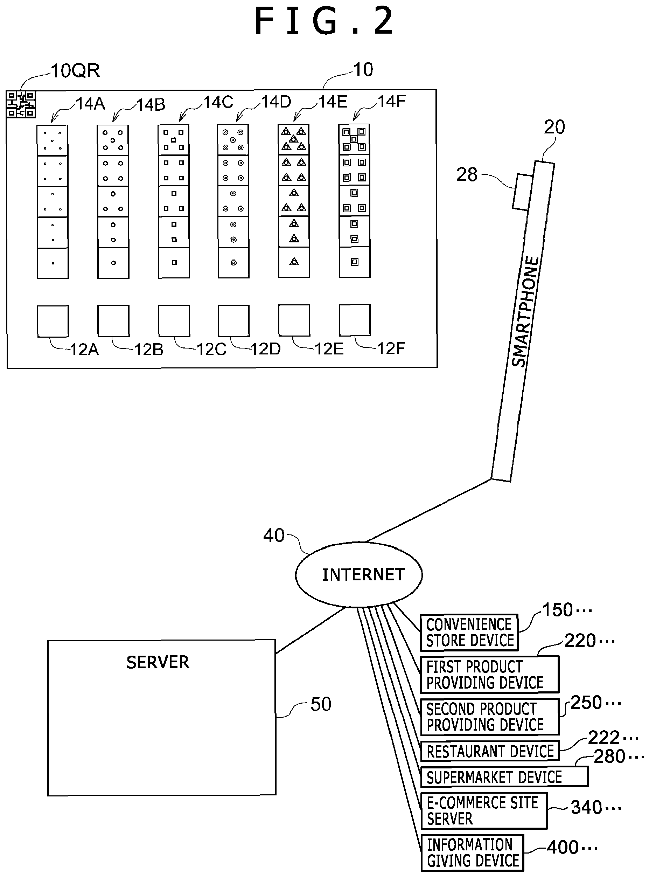

FIG. 2 is a block diagram of the solution providing system 2050. The solution providing system 2050 includes a communication terminal (hereinafter, referred to as a "smartphone") 20, a server device (hereinafter, referred to as a "server") 50, a plurality of convenience store devices 150, a plurality of first product providing devices 220, a plurality of second product providing devices 250, a plurality of restaurant devices 222, a plurality of supermarket devices 280, a plurality of EC site servers 340, and a plurality of information giving devices 400. The smartphone 20 to the information giving device 400 are communicably connected to each other via a network, for example, the Internet 40.

The smartphone 20 images the reagent carrier 10 in which the concentrations of the plurality of reagents 12A, 12B, and 12C have changed due to attachment of the urine 18.

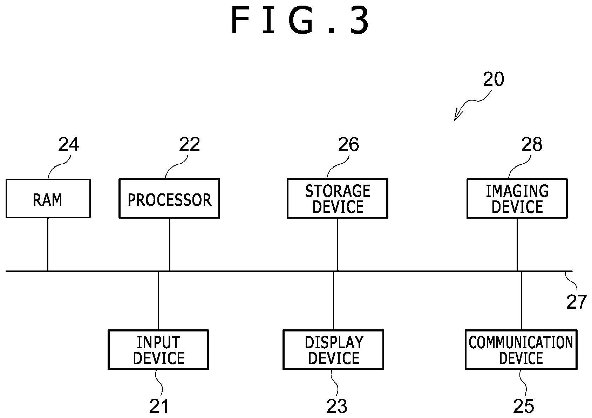

FIG. 3 is a block diagram of the smartphone 20. The smartphone 20 includes a processor 22, a random access memory (RAM) 24, a storage device 26, an imaging device 28, an input device 21, a display device 23, and a communication device 25. The processor 22 to the communication device 25 are communicably connected to each other via a bus 27. The processor 22 controls the RAM24, the storage device 26, the imaging device 28, the input device 21, the display device 23, and the communication device 25.

The display device 23 is an example of a "display unit" and a "presentation unit" of the technology of the present disclosure. The communication device 25 is an example of a "receiving unit", a "transmission unit", and an "access unit" of the technology of the present disclosure.

The processor 22 is a processing device including a digital signal processor (DSP), a central processing unit (CPU), and a graphics processing unit (GPU), and the DSP and the GPU operate under the control of the CPU and are responsible for executing processing related to an image. Here, an example of the processor 54 is a processing device including a DSP, a CPU, and a GPU, but this is merely an example, and the processor 22 may be one or more CPUs and DSPs in which GPU functions are integrated, one or more CPUs and DSPs in which GPU functions are not integrated, or a Tensor Processing Unit (TPU) may be mounted.

The storage device 26 is a nonvolatile storage device that stores various programs, various parameters, and the like. Examples of the storage device 26 include a flash memory (for example, Electrically Erasable and Programmable Read Only Memory (EEPROM)).

The RAM24 is a memory in which information is temporarily stored, and is used as a work memory by the processor 22. Examples of the RAM24 include a dynamic random access memory (DRAM), a static random access memory (SRAM), and the like.

The input device 21 is a keyboard, receives an instruction from a user, and outputs a signal indicating the received instruction to the processor 22.

The display device 23 presents various types of information to the user under the control of the processor 22.

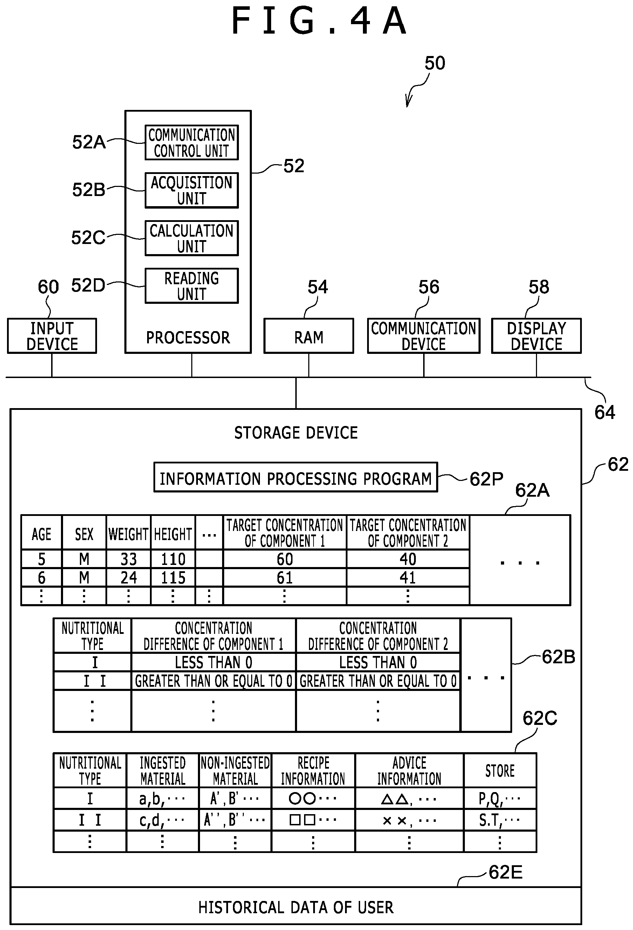

FIG. 4A is a block diagram of the server 50. The server 50 includes a processor 52, an input device 60, a RAM54, a communication device 56, a display device 58, and a storage device 62. The processor 52 to the storage device 62 are communicably connected to each other via a bus 64. The processor 52 controls the input device 60, the RAM54, the communication device 56, the display device 58, and the storage device 62.

The display device 58 is an example of a "presentation unit" of the technology of the present disclosure. The storage device 62 is an example of a "storage unit" of the technology of the present disclosure. The server 50 is an example of a "solution providing device" of the technology of the present disclosure.

The storage device 62 stores an information processing program 62P. The processor 52 reads the information processing program 62P from the storage device 62, and executes the read information processing program 62P on the RAM54 to perform information processing. The processor 52 operates as a communication control unit 54A, an acquisition unit 54B, a calculation unit 54C, and a reading unit 52D according to the information processing program 62P executed on the RAM54, thereby implementing information processing.

The storage device 62 includes a first storage area 62A. In the first storage area 62A, in addition to age, sex, weight, height, and the like, a target concentration of the first component (1), a target concentration of the second component (2), and a target concentration of the third component (3) are stored as attribute data.

The storage device 62 includes a second storage area 62B. In the second storage area 62B, a nutrition type and an aspect of a difference between an actual concentration of each component and the target concentration are stored correspondingly.

Here, the nutrition type is specifically information related to a difference between an actual concentration of each component contained in human urine described later and a target concentration of each component contained in urine of a target living body determined by human attribute data, specifically, information indicating an aspect of the difference. Specifically, the nutrition types include, for example, nutrition types I, II,.... The number of nutrition types is, for example, the following five, but is not limited to five. The difference between the actual concentration and the target concentration is, for example, a difference between the actual concentration and the target concentration. The difference is not limited to the difference, and may be a quotient, a sum, or a product.

### (First Content)

The content of the nutrition type is, for example, the following first content.

Nutrition type I is when any one of the first component (1) to the third component (3) is less than 0 (N). Other components are not considered. Nutrition type I is when only one of protein, coenzyme vitamin, and antioxidant vitamin is in shortage.

Nutrition type II is when the difference of each of the first component (1) to the third component (3) is greater than or equal to 0 (G) and the fourth component (4) is less than 0 (N). The component (5) is not considered. Nutrition Type II is when all of the protein, coenzyme vitamins, and antioxidant vitamins are sufficient, but subtle minerals are insufficient.

Nutrition type III is when the difference of each of the first component (1) to the fourth component (4) is greater than or equal to 0 (G) and the fifth component (5) is less than 0 (N). Nutrition Type III is when all of the protein, coenzyme vitamins, antioxidant vitamins, and subtle minerals are sufficient but the multimeric minerals are insufficient.

Nutrition type IV is when the difference of each of the first component (1) to the fifth component (5) is greater than or equal to 0 (G) and the sixth component (6) is less than 0 (N). Nutrition Type IV is when all of the protein, coenzyme vitamins, antioxidant vitamins, subtle minerals, and multimeric minerals are sufficient but the pH is insufficient.

Nutrition type V is when the difference of each of the first component (1) to the sixth component (6) is greater than or equal to 0 (G). Nutrition type V is when all of protein, coenzyme vitamins, antioxidants vitamins, subtle minerals, macro-minerals, and pH are sufficient.

### (Second Content)

The content of the nutrition type is not limited to the first content. For example, the nutrition type may have the following second content:
Nutrition type I is when the difference of only any one of the first component (1), the third component (3), and the fourth component (4) is less than 0. Other components are not considered.
Nutrition type II is when the difference of each of the first component (1), the third component (3), and the fourth component (4) is greater than or equal to 0 (G) and the second component (2) is less than 0 (N). Other components are not considered.
Nutrition type III is when the difference of each of the first component (1) to the fourth component (4) is greater than or equal to 0 (G) and the fifth component (5) is less than 0 (N). The component (6) is not considered.
Nutrition type IV is when the difference of each of the first component (1) to the fifth component (5) is greater than or equal to 0 (G) and the sixth component (6) is less than 0 (N).
Nutrition type V is when the difference of each of the first component (1) to the sixth component (6) is greater than or equal to 0 (G).

### (Third Content)

The content of the nutrition type is not limited to the first content and the second content. For example, the nutrition type may have the following third content.

Nutrition type I is when all of the first component (1) to the fifth component (5) are not less than 0 (0 or more (G)). The component (6) is not considered.

Nutrition type II is when the first component (1) or the second component (2) is less than 0 (N). Other components are not considered.

Nutrition type III is when the first component (1) and the second component (2) are greater than or equal to 0 (G) and the third component (3) is less than 0 (N). Other components are not considered.

Nutrition type IV is when the difference of each of the first component (1) to the third component (3) is greater than or equal to 0 (G) and the fourth component (4) is less than 0 (N). Other components are not considered.

Nutrition type V is when the difference of each of the first component (1) to the fourth component (4) is greater than or equal to 0 (G) and the fifth component is less than 0 (N). The sixth component (6) is not considered.

The storage device 62 includes a third storage area 62C. In the third storage area 62C, each nutrition type, information on a solution for eliminating the difference in the nutrition type, and information on a store of a product for achieving the solution are stored in correspondence.

The solution information includes an ingested material (food and drink) recommended corresponding to the nutrition type, a non-ingested material not recommended corresponding to the nutrition type, recipe information of the ingested material, and advice information of a life aspect corresponding to the nutrition type. The advice information is generated using an artificial intelligence tool, for example, ChatGPT.

Here, for example, information of a nutrition type I solution will be described. Examples of the information of the ingested material (food and drink) include a (for example, rice cooked with taro), b (for example, Nanbanzuke of vegetables and blue-green fish), c, d, and the like. As described above, the ingested material information includes an intake amount for a certain period (for example, per day) in addition to the product name (rice cooked with taro, or the like). Examples of the information on non-ingested materials include A' (diet soda), B' (fruit syrup), and.... There is recipe information of each of foods a, b, and.... Advice information includes ΔΔ (walking more than 10,000 steps on average per day) and.... The advice information further includes "there is a possibility of ∘∘ disease in this state" and the like.

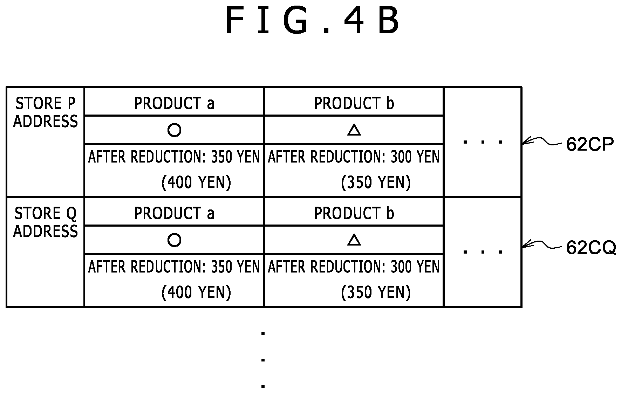

As illustrated in FIG. 4B, the information of the stores includes information 62CP, 62CQ,... of each store where the ingested material can be obtained. For example, when the stores from which the ingested materials a and b can be obtained are a store P and a store Q, the information 62CP of the store P includes the name of the store P and the address of the website of the store P. The information 62CP of the store P includes a product name "a" of the ingested material a, a photograph (illustrated as "o" in FIG. 4B) of the ingested material a, a price after reduction of the ingested material of 350 yen, and a price before reduction of the ingested material of 400 yen. The information 62CP of the store P includes a product name "b" of the ingested material b, a photograph (illustrated as "Δ" in FIG. 4B) of the ingested material b, a price after reduction of the ingested material of 300 yen, and a price before reduction of the ingested material of 350 yen. In the information 62CQ of the store Q, information similar to the information 62CP of the store P is stored. For example, it is assumed that the store P is a real store (convenience store) and the store Q is an electronic commerce (EC) site.

The storage device 62 includes a fourth storage area 62D. In the fourth storage area 62D, history data of each user is stored.

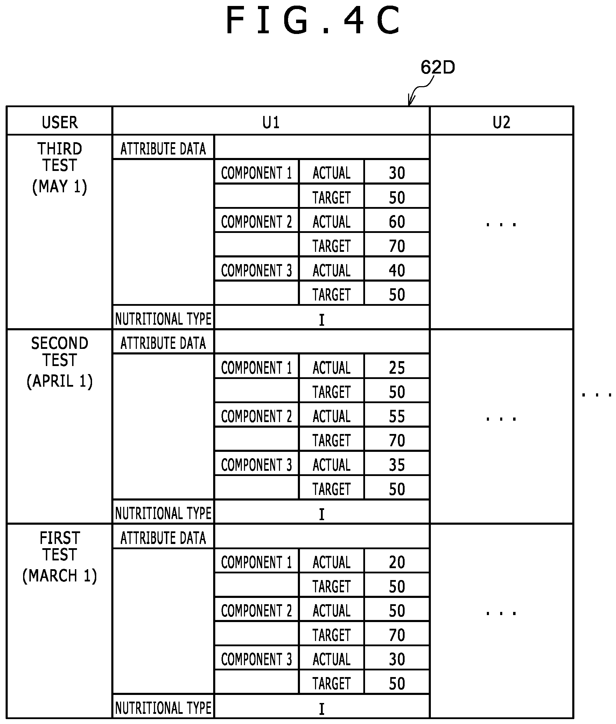

FIG. 4C is a diagram illustrating the history data of the user. In the history data of the user, attribute data and a test result at the time of each test of each user are stored. The test is to calculate the above aspect of the difference using an image of the reagent carrier 10 attached to the urine 18. The test results are the actual concentration, target concentration, and nutrition type of each component.

For example, history data of a user U1 will be described. The user U1 has performed the test three times so far. The history data of the user U1 includes a date of the first test (March 1), attribute data, a specific test result, and a nutrition type I. Specific test results include actual concentration of the first component (1): 20% and target concentration thereof: 50%, actual concentration of the second component (2): 50% and target concentration thereof: 70%, and actual concentration of the third component: 30% and target concentration thereof: 50%.

Data of items similar to those in the first test is stored in each of the second and third tests.

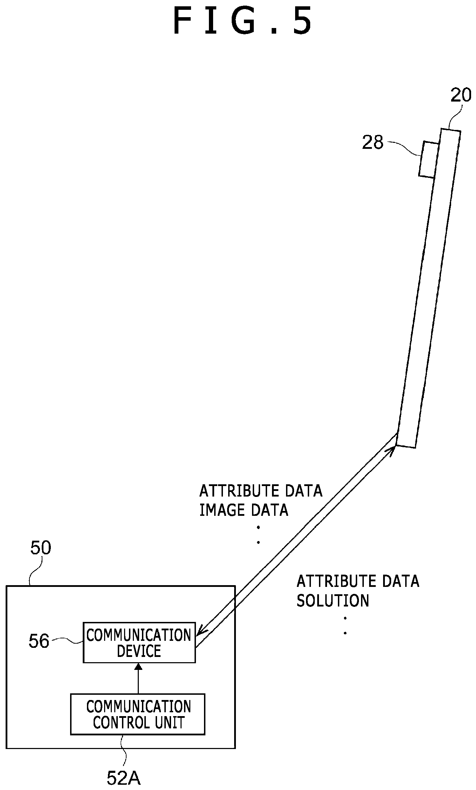

FIG. 5 is a diagram illustrating processing contents of a communication control unit 52A. The communication control unit 52A controls the communication device 56 to receive the attribute data and the image data from the smartphone 0, and transmits the information on the nutrition type, the solution, and the like to the smartphone 20.

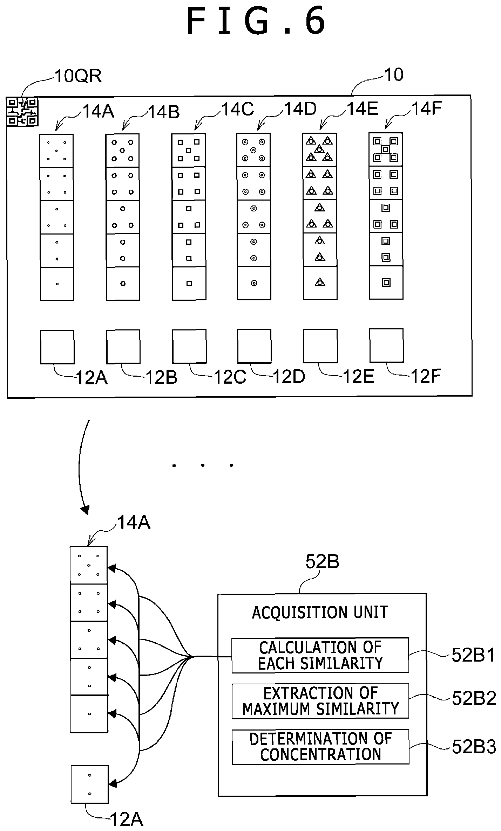

FIG. 6 is a diagram illustrating processing contents of an acquisition unit 52B. The acquisition unit 52B acquires the concentration of each of the six components of the human urine 18. In Step 54B1, the acquisition unit 52B compares, by image matching, each image of the reagents 12A to 12F to which the urine 18 is attached and reacted to develop color with each of the plurality of images 14A to 14F of each of the plurality of concentrations of each of the first component (1) to the sixth component (6) of the urine 18, and calculates similarity. Specifically, for example, the acquisition unit 52B calculates each similarity between the image of the reagent 12A and each of the plurality of images 14A. The acquisition unit 52B extracts the maximum similarity in Step 54B2, and extracts an image corresponding to the maximum luminance value among the plurality of images 14A to determine the concentration in Step 54B3.

As another method of obtaining the similarity, for example, a difference between the maximum luminance value in the image of the reagent 12A and the maximum luminance value of each of the plurality of images 14A is calculated. The smaller the difference, the higher the similarity. The similarity is not limited to the maximum luminance value of each image, and may be obtained by calculating a difference between average values of luminance of the images to be compared. In Step 54B2, the acquisition unit 52B extracts the maximum luminance value among the similarities between the image of the reagent 12A and each of the plurality of images 14A. In Step 54B3, the acquisition unit 52B extracts an image corresponding to the maximum luminance value among the plurality of images 14A and determines the concentration. The acquisition unit 52B similarly processes each of the other second component (2) and third component (3).

As described above, for example, the actual concentration of the first component (1) is 30%, the actual concentration of the second component (2) is 60%, and the actual concentration of the third component (3) is 40%.

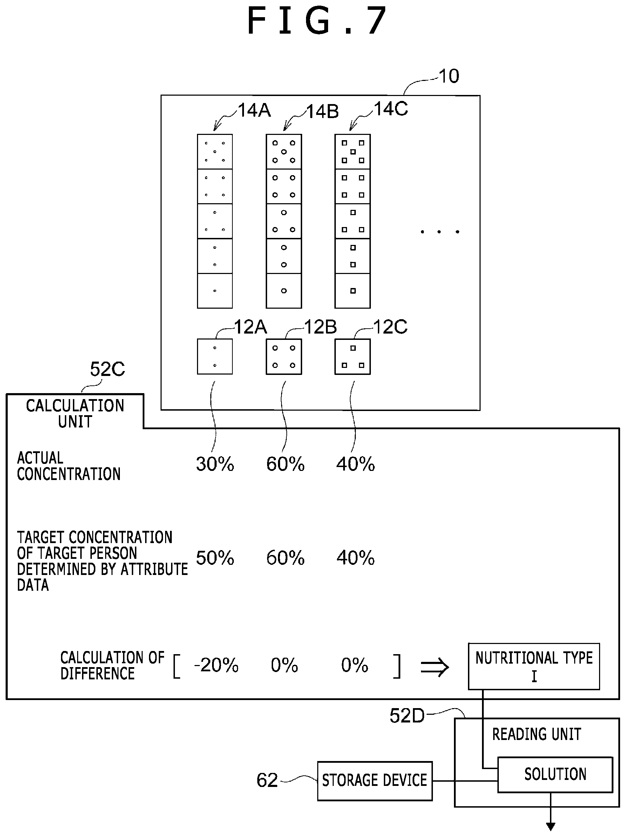

FIG. 7 is a diagram illustrating processing contents of the calculation unit 52C and the reading unit 52D. The calculation unit 52C calculates a difference between an actual concentration of each component and a target concentration of a target person determined by the attribute data. Specifically, 30% (actual concentration) - 50% (target concentration) = -20% is calculated for the first component (1), 60% (actual concentration) - 60% (target concentration) = 0% is calculated for the second component (2), and 40% (actual concentration) - 40% (target concentration) =0% is calculated for the third component (3). The difference is also calculated for the other fourth component (4) to sixth component (6).

Next, the calculation unit 52C determines the nutrition type from the aspect of the difference of each component as follows.

### (When Content of Nutrition Type Is First Content)

First, the calculation unit 52C determines the difference of the first component (1). When the difference is less than 0 (N), it is defined as the nutrition type I. Next, when the difference of the first component (1) is 0 or more (G) and the second component (2) is less than 0 (N), the calculation unit 52C determines the first component (1) as the nutrition type I. Next, when the difference of each of the first component (1) and the second component (2) is 0 or more (G) and the third component (3) is less than 0 (N), the calculation unit 52C sets the type as nutrition type I.

Next, when the difference of each of the first component (1) to the third component (3) is 0 or more (G) and the fourth component (4) is less than 0 (N), it is defined as the nutrition type II.

Next, when the difference of each of the first component (1) to the fourth component (4) is 0 or more (G) and the fifth component (5) is less than 0 (N), it is defined as nutrition type III.

Next, when the difference of each of the first component (1) to the fifth component (5) is 0 or more (G) and the sixth component (6) is less than 0 (N), it is defined as the nutrition type IV.

Finally, when the difference of each of the first component (1) to the sixth component (6) is 0 or more (G), it is defined as the nutrition type V.

### (When Content of Nutrition Type Is Second Content)

First, when the difference of the first component (1) is less than 0, the calculation unit 52C sets the type as the nutrition type I. Next, when the difference of the first component (1) is 0 or more (G) and the third component (3) is less than 0 (N), the calculation unit 52C sets the type as the nutrition type I. Next, when the difference of each of the first component (1) to the third component (2) is 0 or more (G), and the fourth component (4) is less than 0 (N), the calculation unit 52C sets the type as the nutrition type I.

Next, when the difference of each of the first component (1), the third component (3), and the fourth component (4) is 0 or more (G), and when the second component (2) is less than 0 (N), the calculation unit 52C sets the type as the nutrition type II.

Next, when the difference of each of the first component (1) to the fourth component (4) is 0 or more (G) and the fifth component (5) is less than 0 (N), the calculation unit 52C sets the type as the nutrition type III.

Next, when the difference of each of the first component (1) to the fifth component (5) is 0 or more (G) and the sixth component (6) is less than 0 (N), the calculation unit 52C sets the type as the nutrition type IV.

Finally, when the difference of each of the first component (1) to the sixth component (6) is 0 or more (G), the calculation unit 52C sets the type as the nutrition type V.

### (When Content of Nutrition Type Is Third Content)

First, when all of the first component (1) to the fifth component (5) are not less than 0 (0 or more (G)), the calculation unit 52C sets the type as the nutrition type I. The sixth component (6) is not considered.

Next, when the aspect of the difference of each component is not the nutrition type I and the first component (1) or the second component (2) is less than 0 (N), the calculation unit 52C sets the type as the nutrition type II.

Next, when the aspect of the difference of each component is not the nutrition type II, the first component (1) and the second component (2) are 0 or more (G), and the third component (3) is less than 0 (N), the calculation unit 52C sets the type as the nutrition type III.

Next, when the aspect of the difference of each component is not the nutrition type III, the difference of each of the first component (1) to third component (3) is 0 or more (G), and the fourth component (4) is less than 0 (N), the calculation unit 52C sets the type as the nutrition type IV.

Finally, when the aspect of the difference of each component is not the nutrition type IV, the difference of the first component (1) to the fourth component (4) is 0 or more (G), and the fifth component is less than 0 (N), the calculation unit 52C sets the type as the nutrition type V.

When the nutrition type is determined as described above, the reading unit 52D reads the solution information and the store information corresponding to the nutrition type from the third area 62C of the storage device 62.

Configurations of the plurality of convenience store devices 150, the plurality of first product providing devices 220, the plurality of second product providing devices 250, the plurality of restaurant devices 222, the plurality of supermarket devices 280, the EC site server 340, and the information giving device 400 will be described later.

### (Operation Example of Smartphone 20 and Server 50)

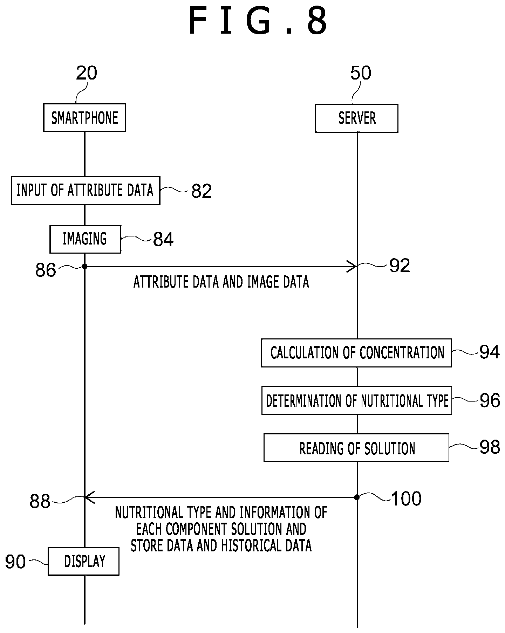

FIG. 8 is a timing chart illustrating an operation example of the smartphone 20 and the server 50.

The user inputs the attribute data via the input device 21 of the smartphone 20. The attribute data includes a name, age, sex, weight, height, frequency of exercise, presence or absence of pregnancy, and a target value of each component. In Step 82, the smartphone 20 receives the input of the attribute data.

The user inputs the reagent carrier 10 into the container 16 in which the urine 18 is stored. The user images the reagent carrier 10 with the imaging device 28 within a predetermined period (for example, 10 seconds) from the input.

The smartphone 20 has a function of operating the imaging device 28 to capture an image when a predetermined period (for example, 10 seconds) has elapsed since the timer was turned on. Therefore, when the user inputs the reagent carrier 10 into the container 16 storing the urine 18, the user turns on the timer, and positions the reagent carrier 10 in which the reagents 12A to 12C adhere to the urine 18 in the imaging area of the imaging device 28. When a predetermined period (for example, 10 seconds) has elapsed since the timer was turned on, the imaging device 28 operates, and the imaging device 28 images the reagent carrier 10.

In Step 84, the smartphone 20 receives image data of the reagent carrier 10 obtained by imaging the reagent carrier 10 by the imaging device 28.

In Step 86, the communication device 25 transmits the attribute data and the image data of the reagent carrier 10 to the server 50 (see FIG. 5).

In the server 50, in Step 92, under the control of a communication processing unit 52A, the communication device 56 receives the attribute data and the image data of the reagent carrier 10.

As described above, the attribute data includes a target value of each component. However, it may be difficult for the user to input the target value of each component. The user may not input the target value of each component. When the target value of each component is not included in the attribute data in this manner, the acquisition unit 52B of the server 50 transmits an inquiry message such as "is there a body shape do you want?" to the smartphone 20 via the communication device 56.

The communication device 25 of the smartphone 20 receives the message, and the display device 23 displays the message. The user inputs a response message such as "I want to have a slimmer body shape" or "I want to have a muscular body shape" via the input device 21, for example. The communication device 25 transmits the response message to the server 50.

The acquisition unit 52B of the server 50 receives the response message via the communication device 56 and sets the target concentration according to the content of the response message. For example, when it is desired that the content of the response message becomes a "slimmer body shape", a value obtained by increasing a standard target concentration value determined from the age, gender, height, and weight by 3%, for example, is set as the target value of each component.

In this manner, the acquisition unit 52B sets the target concentration of each component on the basis of the response to the inquiry to the user.

The target concentration of each component may be determined by age, sex, height, and weight. In this case, when the contents of age, sex, height, and weight change, the target concentration also changes. For example, when the weight decreases by a certain amount, the target concentration of each component also changes by a certain amount.

In Step 94, as described above, the acquisition unit 52B calculates the actual concentration of each component (see FIG. 6). In Step 96, the calculation unit 52C determines the nutrition type (see FIG. 7). In Step 98, the reading unit 52D reads the solution information and the store information from the third area 62C of the storage device 62 (see FIG. 7). In Step 100, under the control of the communication processing unit 52A, the communication device 56 transmits the nutrition type, the information on the concentration of each component (actual concentration, target concentration, and difference thereof), the solution information, and the store information to the smartphone 20 (see FIG. 5).

As described above, the QR code 10QR including information for identifying the shop of the reagent carrier 10 is printed on the reagent carrier 10. From the portion of the QR code 10QR in the image data, the server 50 grasps from which shop the reagent carrier 10 has been purchased, and manages the number of sold reagent carriers 10 for each shop.

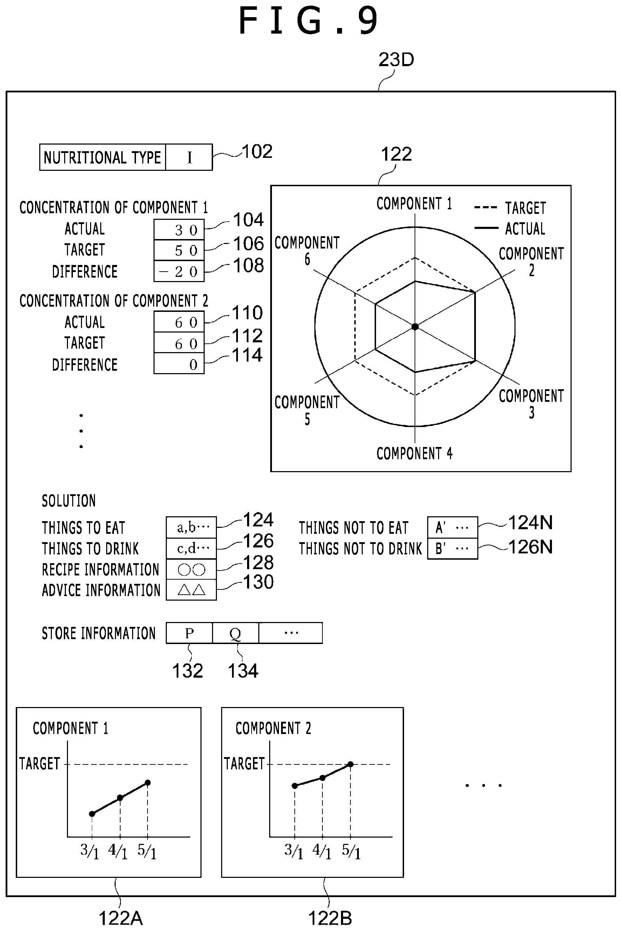

In the smartphone 20, in Step 88, the communication device 25 receives the nutrition type, the information on the concentration of each component, the information on the solution, and the information on the store. In Step 90, the display device 23 displays the nutrition type, information on the concentration of each component, information on the solution, and information on the store as illustrated in FIG. 9. As illustrated in FIG. 9, the display device 23 displays the following information on a display screen 23D.

The display device 23 displays "I" as the nutrition type in the area 102, for example.

For the concentration of the component (1), the display device 23 displays actual concentration: 30 in the area 104, target concentration: 50 in the area 106, and difference: -20 in the area 108. For the concentration of the component (2), the display device 23 displays the actual concentration: 60 in the area 110, the target concentration: 70 in the area 112, and the difference: -10 in the area 114. The display device 23 similarly displays the actual concentration, the target concentration, and the difference for the concentrations of the other components (3) to (6).

The display device 23 displays the actual concentrations and the target concentrations of the components (1) to (6) in the area 122 as a radar chart. For example, the target concentration is indicated by a dotted line, and the actual concentration is indicated by a solid line.

The display device 23 displays, as the solution, things to eat: a, b... in the area 124, things to drink: c, d... in the area 126, recipe information: ∘∘ on things to eat in the area 128, and advice information: ΔΔ in the area 130. The display device 23 displays, as a solution, things not to eat: A'... in the area 124N, and things not to drink: B'... in the area 126N.

The display device 23 displays the information 62CP on the store P (see FIG. 4B) and the information 62CQ on the store Q (see FIG. 4B) in the area 132 as the store information.

The display device 23 displays a temporal change of the actual concentrations of the components (1) to (6) in the areas 122A, 122B,... by a line graph (horizontal axis: test date, vertical axis: concentration value). In the line graph, the target concentrations of the components (1) to (6) are indicated by dotted lines. This makes it easy for the user to understand the temporal change in the actual concentration, and it is also possible to indicate whether or not the concentration is approaching the target concentration.

### (Effects)

As described above, in the present embodiment, the actual concentration of each component in human urine can be grasped, and the nutrition type can be directly grasped from human urine.

In addition, the present embodiment can notify the user of the solution information and the store information according to the nutrition type, and can provide a benefit that the user can make an effort to bring the actual concentration close to the target concentration.

### (Operation Example of Smartphone 20 and Convenience Store Device 150 in Convenience Store)

Next, an operation example of the smartphone 20 at the convenience store and the device (hereinafter, referred to as a "convenience store device")150 arranged at the convenience store will be described.

The convenience store device 150 is an example of a "payment amount calculation device" and a "point addition device" of the technology of the present disclosure. The technology of the present disclosure is not limited to a device of a convenience store, and may be a device of another retail store.

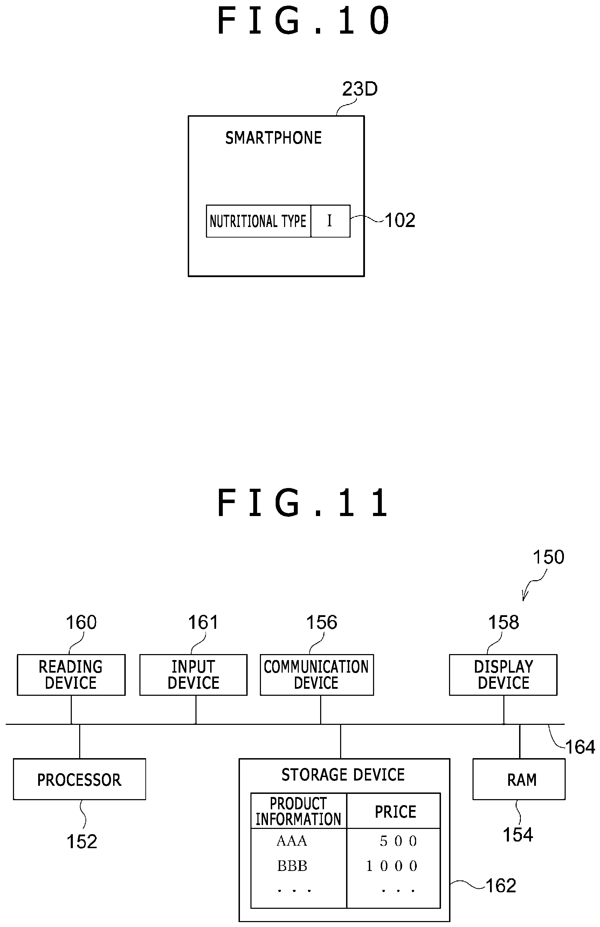

FIG. 10 is a diagram illustrating a screen 23D that displays the nutrition type of the display device 23 of the smartphone 20. As described above, the smartphone 20 receives the nutrition type (for example, nutrition type I) from the server 50 (see Step 88 in FIG. 8).

As illustrated in FIG. 10, the smartphone 20 displays the nutrition type (for example, nutrition type I) on a screen 23D of the display device 23. As a result, the store clerk of the convenience store can understand that the user has performed the above test.

FIG. 11 is a block diagram of the convenience store device 150. The convenience store device 150 includes a processor 152, a RAM154, a storage device 162, a reading device 160, an input device 161, a communication device 156, and a display device 158. The processor 152 to the display device 158 are communicably connected to each other by a bus 164. The processor 152 controls the RAM154, the storage device 162, the reading device 160, the input device 161, the communication device 156, and the display device 158.

The storage device 162 stores the product information and the price corresponding to each other. For example, the product information: AAA and the price: 500 yen are correspondingly stored. The product information: BBB and the price: 1000 yen are correspondingly stored.

The reading device 160 is an example of a "reading unit" of the technology of the present disclosure. The processor 152 is an example of a "payment amount calculation unit" and an "addition unit" of the technology of the present disclosure.

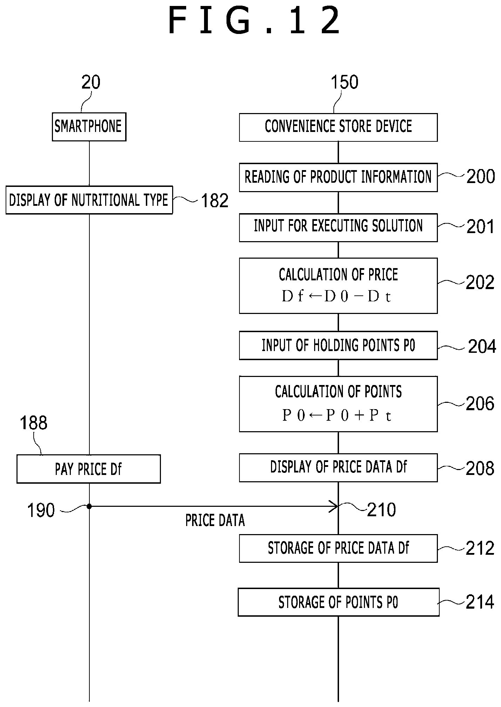

FIG. 12 is a timing chart illustrating an operation example of the smartphone 20 and the convenience store device.

Information (see FIG. 4B) such as stores P and Q is displayed on the screen 23D of the display device 23 of the smartphone 20. Therefore, the user who has performed the above test knows that the product a or the like corresponding to the nutrition type I can be acquired at the store P or the like, and the product a or the like can be purchased at a reduced price. Therefore, in order to realize the solution, for example, the user visits the store P (convenience store) and delivers the product a to the store clerk to purchase the product a. In addition, the user provides the store clerk with a point card (an example of a point carrier). At that time, in Step 182, in accordance with the user's operation, the display device 23 displays the nutrition type (for example, nutrition type I) on the screen 23D as illustrated in FIG. 10 as illustrated in FIG. 10. As a result, the store clerk of the convenience store can understand that the user has performed the above test.

In Step 200, the reading device 160 of the convenience store device 150 reads the product information attached to the product a. In Step 210, according to the operation of the store clerk, the input device 161 inputs the execution of the solution indicating that the user intends to achieve the solution by purchasing the product a. When the execution of the solution is input, the convenience store device 150 is designated to reduce the price and to add points. However, when the user has not performed the above test and the nutrition type is not displayed on the screen 23D of the display device 23, the store clerk does not input execution of the solution. Therefore, there will be no reduction in the price or addition in points.

Once the execution of the solution is input as described above, the processor 152 performs reduction calculation of the price in Step 202. Specifically, the processor 152 reads the price D0 of the product of AAA from the storage device 162 based on the product information, for example, AAA, and calculates the price obtained by subtracting the reduced price Dt from the price D0 as the final price Df.

In Step 204, the reading device 160 reads the holding point P0 from the point card, and in Step 206, the processor 152 performs the addition of points. Specifically, the processor 152 calculates a value obtained by adding an additional point Pt (point obtained by adding point corresponding to product price D0 and point for execution of solution) to the holding point P0 as the newly holding point P0.

In Step 208, the display device 158 displays price data Df. As a result, the user can know the reduced price as the price of the product a.

The smartphone 20 performs payment processing of the price Df, and in Step 190, the communication device 25 transmits the price data. In Step 210, the communication device 156 of the convenience store device 150 receives the price data, in Step 212, stores the price data in the storage device 162, and in Step 214, the communication device 156 stores the newly holding point in the point card. The store clerk returns the point card in which the new point is stored to the user.

As described above, the convenience store device 150 according to the present embodiment can provide a benefit of assisting (the reduction in price and the addition of points) the execution of the solution to the user who performs the above test and intends to execute the solution.

The convenience store device 150 may acquire information on the number of above tests of the user and the temporal change of the actual concentration of each component from the server 50, and increase each of the reduced price Dt and the additional point Pt by a certain value according to at least one of the number of tests and the temporal change.

### (Operation Example of Smartphone 20 and First Product Providing Device 220)

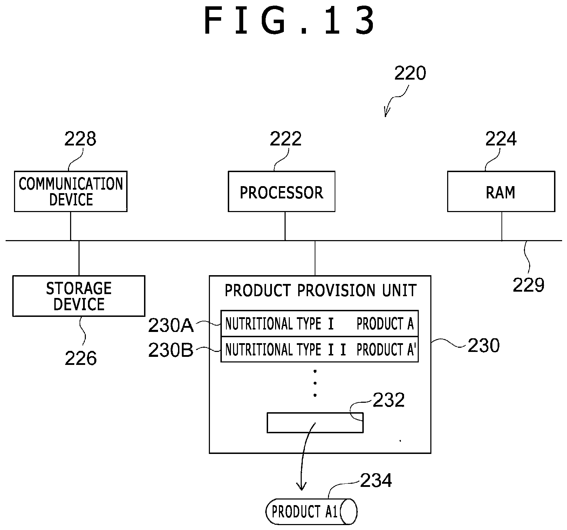

FIG. 13 is a block diagram of first product providing device 220. The first product providing device 220 includes a processor 222, a RAM224, a communication device 228, a storage device 226, and a product providing unit 230. The processor 222 to the product providing unit 230 are communicably connected to each other by a bus 229. The processor 222 controls the RAM224, the communication device 228, the storage device 226, and the product providing unit 230.

The product providing unit 230 can provide the product corresponding to each nutrition type. For example, the product providing unit 230 discharges a product A corresponding to the nutrition type I and discharges a product A' corresponding to the nutrition type II from a discharge port 232. Note that the first product providing device 220 provides a can of a nutritional drink corresponding to each nutrition type as a product. The technology of the present disclosure is not limited to the can, and may be a supplement or the like.

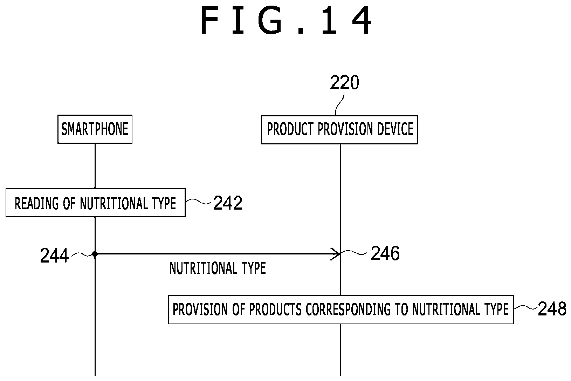

FIG. 14 is a timing chart illustrating an operation example of the smartphone 20 and the first product providing device 220.

In the smartphone 20, in Step 242, the storage device 26 reads the nutrition type after following the user's operation on the input device 21, and in Step 242, the communication device 25 transmits the nutrition type (for example, nutrition type I) to the first product providing device 220.

In the first product providing device 220, in Step 246, the communication device 228 receives data of the nutrition type, and in Step 248, the product providing unit 230 provides the product corresponding to the nutrition type. For example, the product A corresponding to the nutrition type I is discharged.

As described above, the first product providing device 220 of the present embodiment can provide a benefit of providing the product corresponding to the nutrition type.

The technology of the present disclosure is not limited to the transmission of the nutrition type from the smartphone 20 to the first product providing device 220. For example, the nutrition type displayed by a barcode on the display device 23 of the smartphone 20 may be read by a reading device included in the first product providing device 220, for example, a barcode reader.

### (Operation Example of Smartphone 20 and Second Product Providing Device 250)

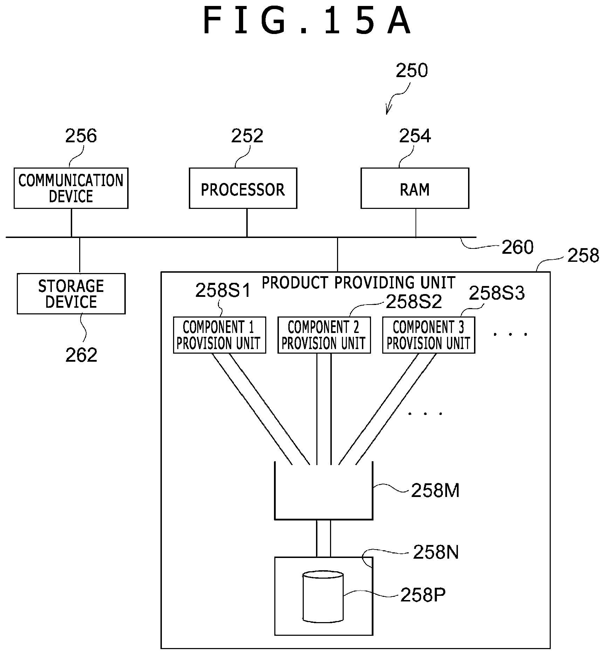

FIG. 15A is a block diagram of the second product providing device 250. The second product providing device 250 includes a processor 252, a RAM254, a communication device 256, a storage device 262, and a product providing unit 258. The processor 252 to the product providing unit 258 are communicably connected to each other by a bus 260. The processor 252 controls the RAM254, the communication device 256, the storage device 262, and the product providing unit 258.

The communication device 256 is an example of an "acquisition unit" of the technology of the present disclosure. The product providing unit 258 is an example of a "manufacturing unit" of the technology of the present disclosure. The processor 252 is an example of a "control unit" of the technology of the present disclosure.

The product providing unit 258 includes a component 1 providing unit 258S1 that provides the component 1, a component 2 providing unit 258S2 that provides the component 2, a component 3 providing unit 258S3 that provides the component 3,....

The components from the component 1 providing unit 258S1 to the component 3 providing unit 258S3... are provided to a mixing unit 258M via a tube, and when components are mixed in the mixing unit 258M and an opening/closing unit 258MO is opened, a mixture (for example, nutritional drink) is discharged to a takeout container 258P at a takeout port 258N via the tube. The user can take the container 258 out of the takeout port 258N to obtain the mixture.

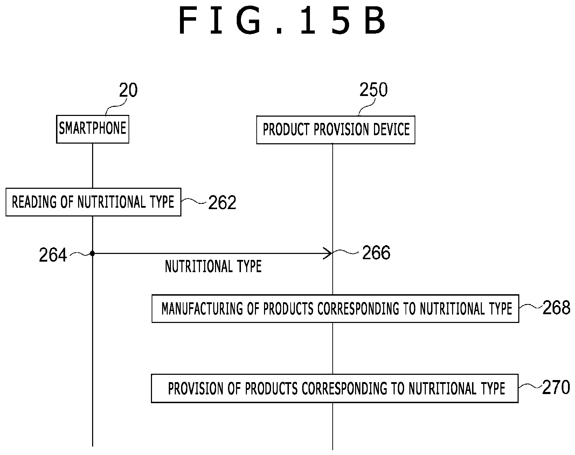

FIG. 15B is a timing chart illustrating an operation example of the smartphone 20 and the second product providing device 250.

In the smartphone 20, in Step 262, the storage device 26 reads the nutrition type after following the user's operation on the input device 21, and in Step 262, the communication device 25 transmits the nutrition type (for example, nutrition type I) to the second product providing device 250.

In the second product providing device 250, in Step 266, the communication device 256 receives the nutrition type data.

In Step 268, processor 252 controls the component 1 providing unit 258S1 to the component 3 providing unit 258S3... such that the components 1 to 3,... having amounts according to the nutrition type I are provided to the mixing unit 258M. From the component 1 providing unit 258S1 to the component 3 providing unit 258S3..., when the opening/closing unit of each providing unit is opened, a certain amount of liquid of each component is discharged (dropped) per unit time. Therefore, when the opening/closing unit of each providing unit is opened for a predetermined time according to the nutrition type, the liquid corresponding to the nutrition type is discharged. The mixing unit 258M mixes the discharged liquids.

In Step 270, the processor 252 opens the opening/closing unit 258MO. As a result, the mixture (for example, nutritional drink) is discharged to the takeout container 258P at the takeout port 258N via the tube. The user can take the container 258 out of the takeout port 258N to obtain the mixture.

As described above, the second product providing device 250 according to the present embodiment can provide a benefit of providing the product (nutritional drink) corresponding to the nutrition type. Note that the product is not limited to the nutritional drink, and may be, for example, curry powder, sprinkled rice, or the like.

The technology of the present disclosure is not limited to the transmission of the nutrition type from the smartphone 20 to the second product providing device 250. For example, the nutrition type displayed by a barcode on the display device 23 of the smartphone 20 may be read by the reading device included in the second product providing device 250, for example, a barcode reader.

### (Operation Example of Restaurant Device 222 and Server 50)

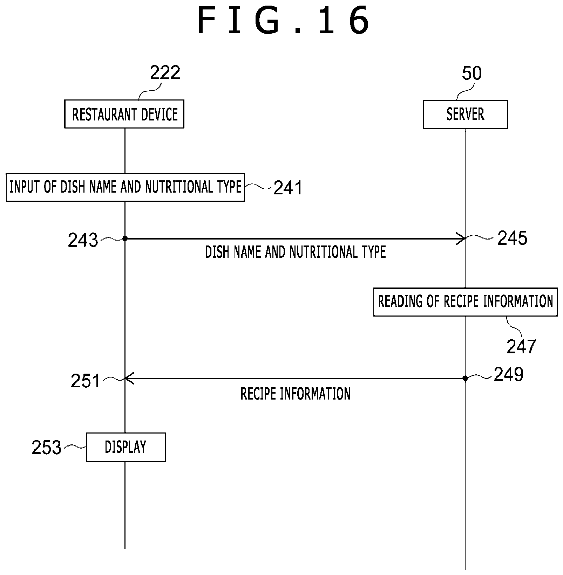

FIG. 16 is a timing chart illustrating an operation example of the restaurant device 222 and the server 50. Note that since the restaurant device 222 has the same configuration as the server 50, the description thereof will be omitted. Note that a configuration similar to that of the server 50 of the restaurant device 222 is denoted by R to be distinguished.

In the restaurant device 222, the user notifies the store clerk of the nutrition type and a dish name, displays the nutrition type and the dish name on the display device 23, and further transmits the nutrition type and the dish name from the smartphone 20 to the restaurant device 222.

In Step 241, the input device 60R inputs the nutrition type and the dish name according to the operation of the restaurant staff. In Step 243, the communication device 56R transmits the nutrition type and the dish name to the server 50.

In the server 50, in Step 245, the communication device 56 receives the nutrition type and the dish name; in Step 247, reads, from the third area 62C of the storage device 62, recipe information corresponding to the nutrition type and the dish name; and in Step 249, the communication device 56 sends the recipe information to the restaurant device 222.

In Step 253, the display device 58R displays the recipe information.

As a result, in the restaurant, a dish corresponding to the nutrition type is made and provided to the user. In this manner, the restaurant device 222 can provide a benefit of providing the user with the dish corresponding to the nutrition type.

### (Operation Example of Smartphone 20 and Supermarket Device 280)

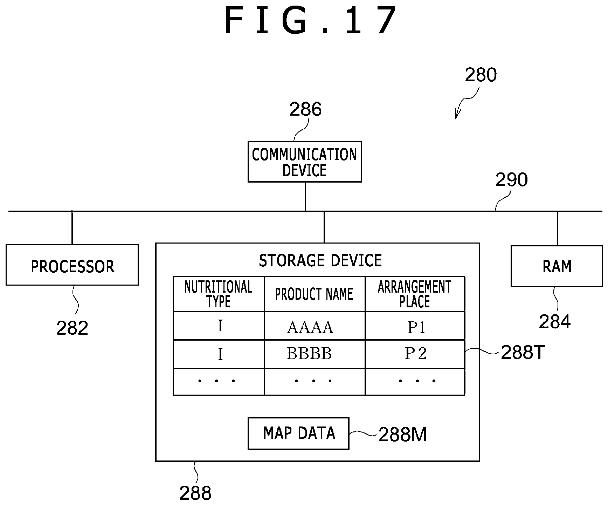

FIG. 17 is a block diagram of the supermarket device 280. The supermarket device 280 includes a processor 282, a RAM284, a communication device 286, and a storage device 288. The processor 282, the RAM284, the communication device 286, and the storage device 288 are communicably connected to each other via a bus 290. The processor 282 controls the RAM284, the communication device 286, and the storage device 288.

The storage device 288 stores a table 288T that correspondingly stores the nutrition type, the name of the product, and the arrangement place, and map data 288M of an area where the product of the supermarket is arranged. In the table 288T, for example, the nutrition type I, the product name: AAAA, and an arrangement place P1 are correspondingly stored.

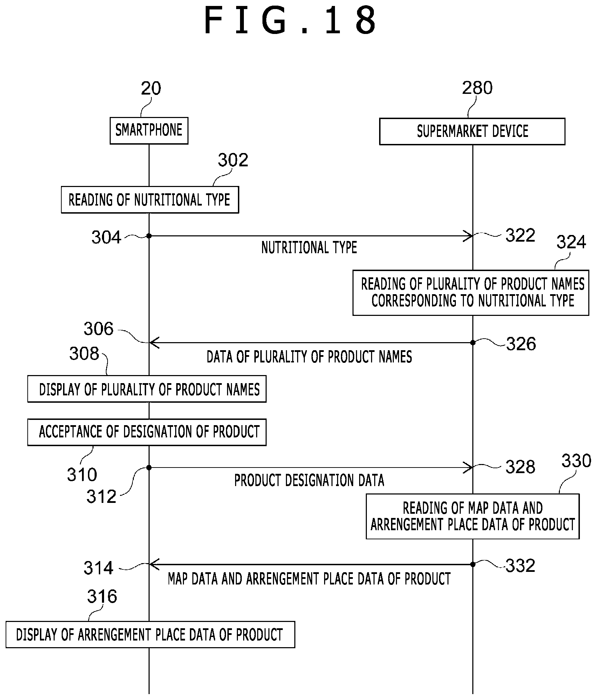

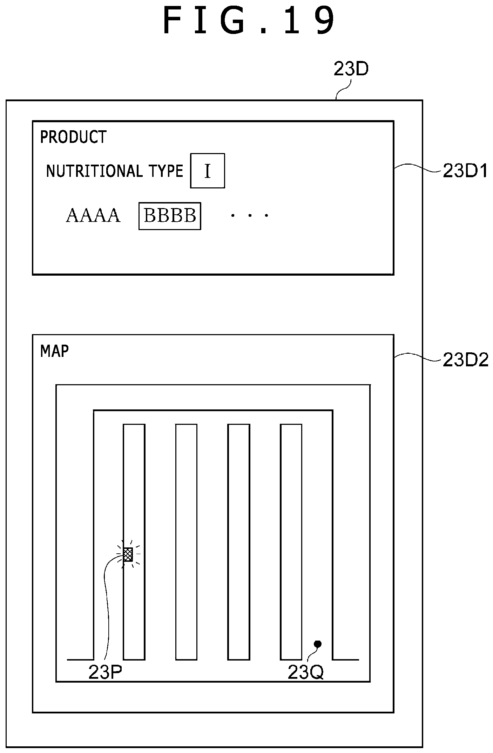

FIG. 18 is a timing chart illustrating an operation example of the smartphone 20 and the supermarket device 280. FIG. 19 is a diagram illustrating the product name and current positions of the designated product and the user in a selling area of a supermarket.

In the smartphone 20, in Step 302, the processor 22 reads the nutrition type from the storage device 26 in accordance with an operation of the user on the input device 21, and in Step 304, the communication device 25 transmits the nutrition type (for example, nutrition type I) to the supermarket device 280.

In the supermarket device 280, in Step 322, the communication device 286 receives the nutrition type I, in Step 324, the processor 282 reads a plurality of product names: AAAA, BBBB,... corresponding to the nutrition type I, and in Step 326, the communication device 286 sends the plurality of product names: AAAA, BBBB,... corresponding to the nutrition type I to the smartphone 20.

In the smartphone 20, in Step 306, the communication device 25 receives a plurality of product names: AAAA, BBBB,... corresponding to the nutrition type I; and in Step 308, the display device 23 displays, in the first area 23D1 of the screen 23D, the plurality of product names: AAAA, BBBB,... corresponding to the nutrition type I, as illustrated in FIG. 19. The user viewing these displays operates the input device 21 to designate (click) the name of the product to be purchased, for example, BBBB, from the product names AAAA, BBBB,... displayed in the first area 23D1. In Step 310, the processor 22 accepts designation of BBBB. In Step 312, the communication device 25 transmits the product designation data (name of BBBB) to the supermarket device 280.

In the supermarket device 280, in Step 328, the communication device 286 receives the designated data of the product, in Step 330, the processor 282 reads the map data 288M and the designated arrangement place P2 of the product from the storage device 288, and in Step 332, the communication device 286 transmits the map data 288M and the designated arrangement place P2 of the product to the smartphone 20.

In the smartphone 20, in Step 314, the communication device 25 receives the map data 288 and the designated arrangement place P1 of the product, and in Step 316, the display device 23 displays, in the second area 23D2 of the screen 23D, the position 23P of the BBBB in the area where the product of the supermarket is arranged, as illustrated in FIG. 19. In the example illustrated in FIG. 19, a position 23Q of the smartphone 20 is also illustrated in the area. Note that the position of the smartphone 20 is detected on the basis of a GPS signal received by a GPS signal receiving unit (not illustrated).

As described above, the supermarket device 280 of the present embodiment can provide a benefit of presenting the position of the product designated by the user in the above area.

The presentation of the position of the product designated by the user in the above area is not limited to display on the screen of the display device. For example, it may be presented by voice.

### (Operation Example of Smartphone 20 and Server 340 of EC Site)

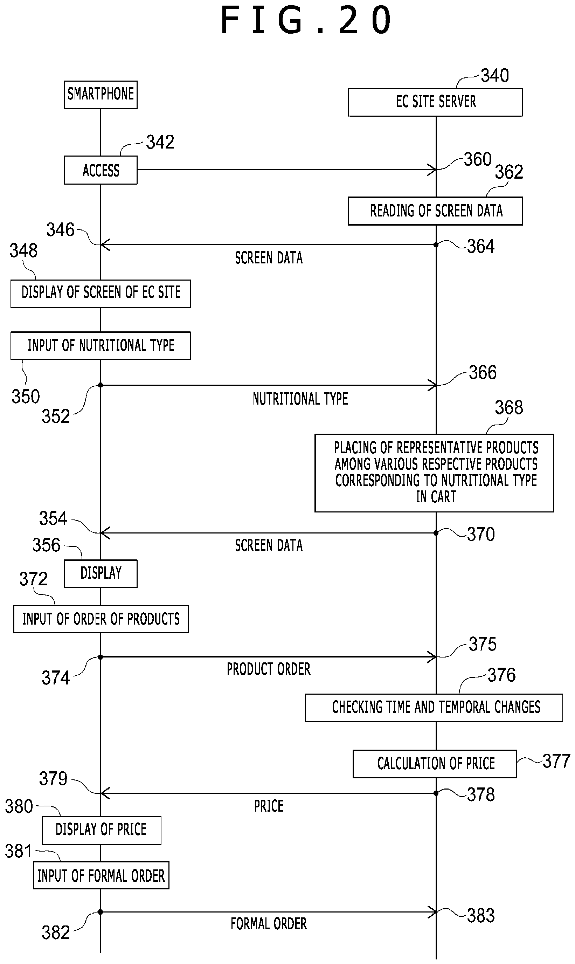

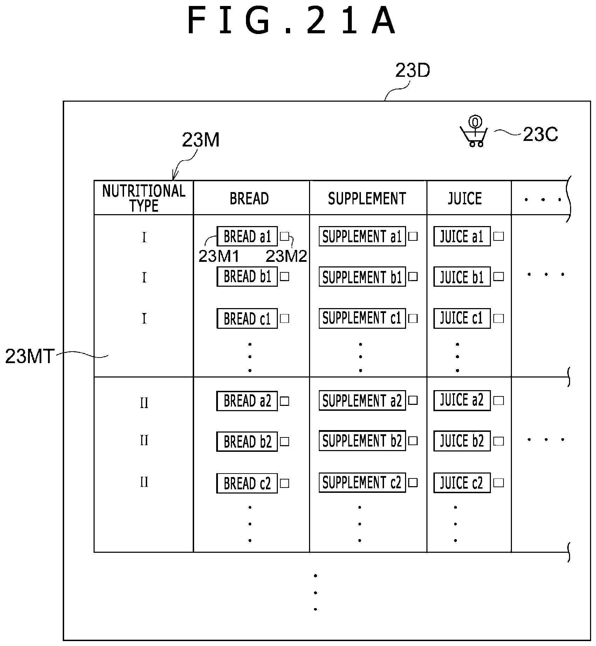

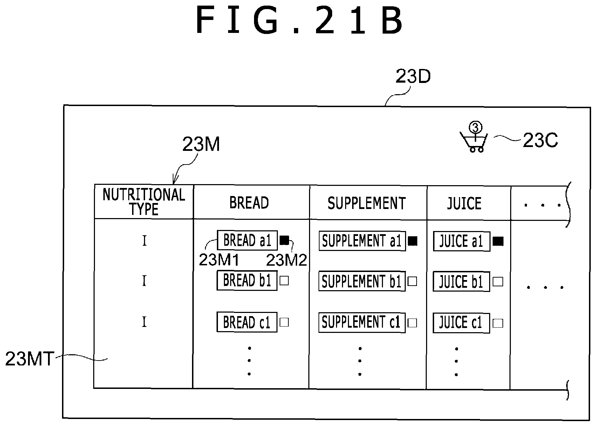

FIG. 20 is a timing chart illustrating an operation example of the smartphone 20 and the server 340 of the EC site. FIG. 21A is a diagram illustrating an order screen of a product of each nutrition type downloaded from the server 340 of the EC site of the smartphone 20. FIG. 21B is a diagram illustrating an order screen illustrating a state in which a representative good in the nutrition type designated by the user is provisionally ordered.

Since the configuration of the server 340 of the EC site is similar to that of the server 50, the description thereof will be omitted. Note that a configuration similar to that of the server 50 of the server 340 of the EC site is denoted by E to be distinguished.

As described above, in the smartphone 20, the display device 23 displays the information on the store on the display screen 23D in Step 90 of FIG. 8 (see FIG. 9). For example, the information 62CQ on the store Q (see FIG. 4B) is displayed. The information 62CQ on the store Q includes the name of the store Q and the address of the website of the store Q.

The user clicks the address of the website of the store Q. In Step 342, the communication device 25 accesses the address.

In the server 340 of the EC site, in Step 360, the communication device 56E receives the access, in Step 362, the processor 52E reads the screen data of the order screen of the product of each nutrition type from the storage device 62E, and in Step 364, the communication device 56E transmits the screen data to the smartphone 20.

In the smartphone 20, in Step 346, the communication device 25 receives screen data, and in Step 348, the display device 58E displays an order screen of a product of each nutrition type in the EC site, as illustrated in FIG. 21A. For example, corresponding to the nutrition type I, individual product names (for example, bread a1) 23M1,... and buttons 23M2,... for a provisional order of the product name 23M1 are displayed for bread. The same applies to other products such as supplements and juices. The same applies to other nutrition types II,....

In Step 350, according to the operation of the user, the input device 21 clicks, for example, a field 23MT of the nutrition type I of the user to input the nutrition type I. In Step 352, the communication device 25 transmits the nutrition type I to the server 340 of the EC site.

In the server 340 of the EC site, the communication device 56E receives the nutrition type I, and in Step 368, the processor 52E creates a screen for placing a representative product among various respective products corresponding to the nutrition type I in a cart 23C, as illustrated in FIG. 21B. For example, in the case of bread, the button 23M2 corresponding to the product name 23M1 is turned on. Similarly, for other supplements and juices, the button corresponding to the product name of the representative product is turned on. In this case, three products are designated in the cart 23C.

In Step 370, the communication device 56E transmits the screen data created in Step 368 to the smartphone 20. In the smartphone 20, in Step 354, the communication device 25 receives the screen data, and in Step 356, the display device 23 displays the screen data. As a result, the user can understand that the bread with the product name 23M1 has been provisionally ordered. It can be understood that other supplements and juices are also provisionally ordered. The user who accepts the above contents turns on a product confirmation button (not illustrated). When it is desired to correct the above content, a button corresponding to another product name is clicked. For example, when the button corresponding to the product name of the bread b1 is clicked, the button 23M2 of the bread a1 is turned off, and the button corresponding to the bread b 1 is turned on. When not ordering the supplement, the button corresponding to the supplement a1 is turned off by clicking on the button. The user who accepts the contents of the screen turns on the product confirmation button (not illustrated). Accordingly, in Step 374, the communication device 25 transmits the data of the order of the confirmed product to the server 340 of the EC site.

In the server 340 of the EC site, in Step 375, the communication device 56E receives the data of the order of the confirmed product, and in Step 376, the communication device 56E receives the number of times of the above test and the temporal change of each concentration of the user from the server 50, and the processor 52E checks the number of times of the above test and the temporal change of each concentration. In Step 377, the processor 52E calculates the price. When the number of times of the above test is one, the price is not discounted. Meanwhile, when the number of times of the above test is plural, the price is discounted by a certain discount amount or a discount amount that increases as the number of times increases. In addition, when the number of times of the above test is plural, when each concentration is improved (when the concentration is close to the target concentration), the price is further discounted in a praising sense. In Step 378, the communication device 56E transmits data of the calculated price to the smartphone 20.

In the smartphone 20, in Step 379, the communication device 25 receives the data of the price, in Step 380, the display device 23 displays the price, and in Step 381, according to the operation of the user, the input device 21 turns on a formal order button. Accordingly, in Step 382, the communication device 25 transmits the data of the formal order to the server 340 of the EC site.

At the server 340 of the EC site, in Step 383, the communication device 56E receives data of the formal order. As a result, the trade of the product is established. Dispatch of the product is made.

As described above, the server 340 of the EC site of the present embodiment can provide a benefit of being able to establish the trade of the product corresponding to the nutrition type. The user can acquire the ordered product.

### (Operation Example of Smartphone 20 and Server 50 for Purchase of Reagent Carrier 10)

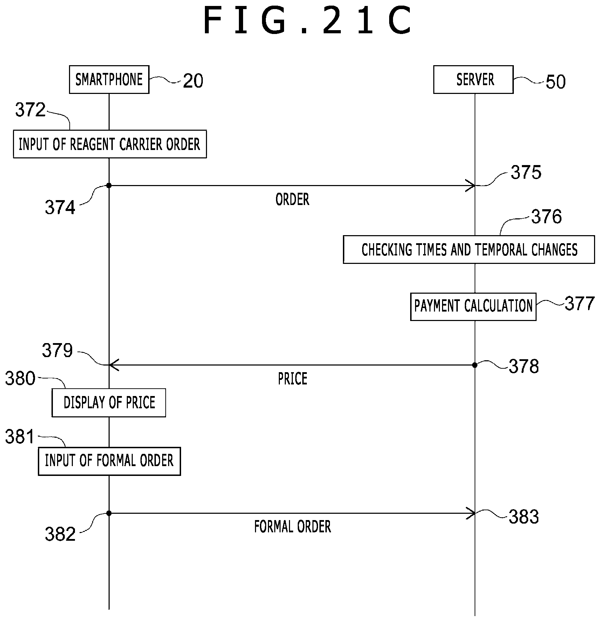

FIG. 21C is a timing chart illustrating an operation example of the smartphone 20 and the server 50 for purchasing the reagent carrier 10.

In the smartphone 20, in Step 372, according to the operation of the user, the input device 21 inputs the order of the reagent carrier 10. In Step 374, data of the order is transmitted to the server 50.

At the server 50, in Step 375, the communication device 56 receives data of the order.

Hereinafter, the processing of calculating the price and the processing of the smartphone 20 and the server 50 of the formal order (Steps 376 to 383) are similar to the processing of Steps 376 to 383 of FIG. 20, and thus the description thereof will be omitted.

### (Information Giving Device 400)

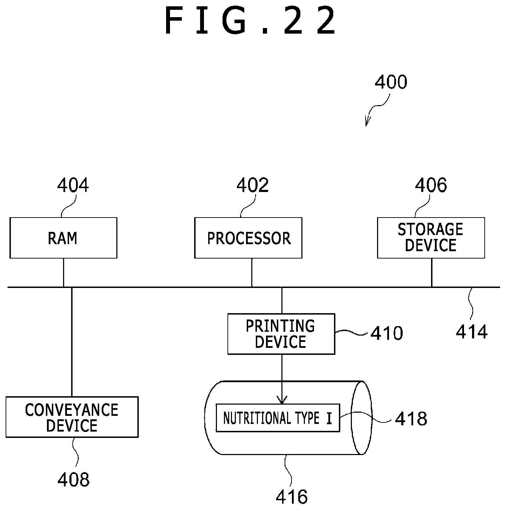

FIG. 22 is a block diagram of the information giving device 400. The information giving device 400 includes a processor 402, a RAM404, a storage device 406, a conveyance device 408, and a printing device 410. The processor 402 to the printing device 410 are communicably connected to each other via a bus 414.

The conveyance device 408 conveys a product packaging 416 to the printing device 410. The printing device 416 prints the nutrition type, for example the nutrition type I, on the product packaging 416 being conveyed.

Since the information giving device 400 prints the nutrition type on the packaging of the product, it is possible to provide a benefit that the user can easily acquire the product for realizing his/her own solution.

The printing device 416 may directly print on the product. Instead of the printing device 410, a sticking device for sticking a seal printed with the nutrition type to the product or the packaging of the product may be provided. The indication is not limited to the character of the nutrition type, and may be a number or a symbol indicating the nutrition type.

### (Shelf 500 of Convenience Store)

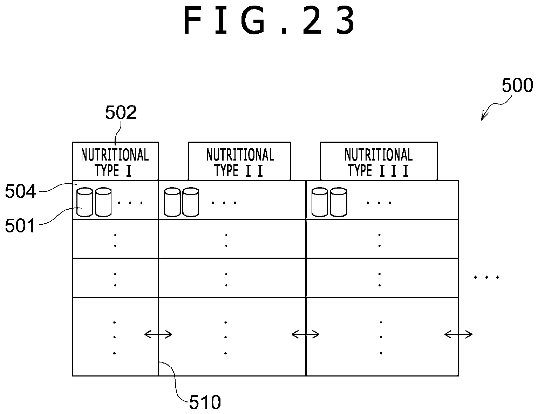

FIG. 23 is a front view illustrating a shelf 500 that is arranged in a convenience store, is partitioned according to each nutrition type, and stores an ingested material (food and drink) recommended to be ingested corresponding to each nutrition type.

In this manner, the shelf 500 is partitioned according to each nutrition type, so that the benefit of facilitating selection and purchase of the ingested material recommended to be ingested corresponding to each nutrition type can be provided.

The shelf 500 is partitioned by a partition plate 510, and the partition plate 510 is movably arranged on the shelf 500 so that the size of the storage area defined by the partition plate 510 is changed.

Each partitioned section is provided with a display unit 501 that displays information indicating the nutrition type.

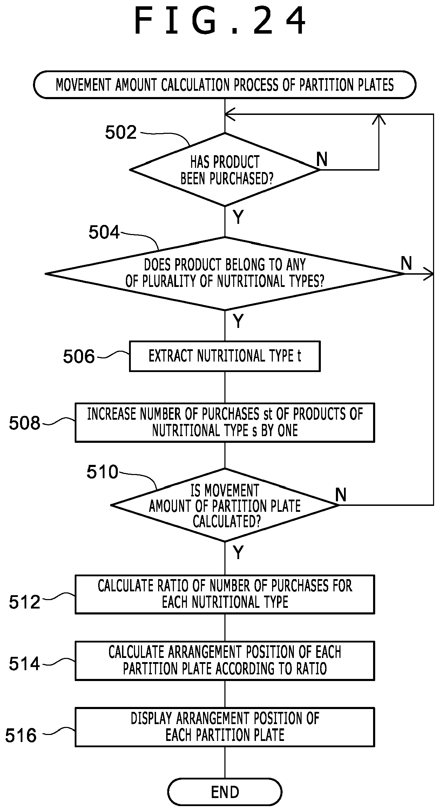

FIG. 24 is a flowchart of partition plate movement amount calculation processing for calculating the movement amount of the partition plate 510 partitioning the storage area of the shelf 500. The movement amount calculation processing of the partition plate is executed by the convenience store device 150.

In Step 502, the processor 152 determines whether the product has been purchased. When the product is not purchased, the processing of Step 502 is repeated. For example, when the storage processing of the price data in Step 212 of FIG. 12 is performed, it is determined that the product has been purchased, and the movement amount calculation processing proceeds to Step 504.

In Step 504, the processor 152 determines whether the purchased product belongs to any of a plurality of nutrition types based on the product information read in Step 200 of FIG. 12. When it is not determined that the purchased product belongs to any of the plurality of nutrition types, the movement amount calculation processing returns to Step 502. When it is determined that the purchased product belongs to any of the plurality of nutrition types, the movement amount calculation processing proceeds to Step 506.

In Step 506, the processor 152 extracts the nutrition type t, and in Step 508, the processor 152 adds 1 to the number of purchases st of the product of the nutrition type t.

In Step 510, it is determined whether or not to calculate the movement amount of the partition plate. When the instruction to calculate the movement amount of the partition plate is not input by the input device 161 according to the user's operation, negative determination is made in Step 510, and the movement amount calculation processing returns to Step 502. When it is instructed to calculate the movement amount of the partition plate, an affirmative determination is made in Step 510, and the movement amount calculation processing returns to Step 512.

In Step 512, the processor 152 calculates a ratio of the number of purchases for each nutrition type, and in Step 514, the processor 152 calculates the arrangement position of each partition plate according to the ratio calculated in Step 512. In Step 516, the display device 158 displays the arrangement position of each partition plate.

As a result, the store clerk of convenience stores arranges the partition plate at the displayed arrangement position. Therefore, since the size of the storage area of each nutrition type of the limited shelf 500 is changed according to the number of purchases of the product of each nutrition type, the other 500 storage areas can be effectively utilized.

The convenience store device 150 is an example of a "movement amount calculation device" of the technology of the present disclosure. The processor 152 is an example of a "counting unit" and a "calculation unit" of the technology of the present disclosure.

### [Modification]

Next, a modification of the technology of the present disclosure will be described.

### (First Modification)

Since a configuration of a first modification is similar to the configuration of the above-described embodiment, the same portions are denoted by the same reference numerals, the description thereof is omitted, and only different portions will be described.

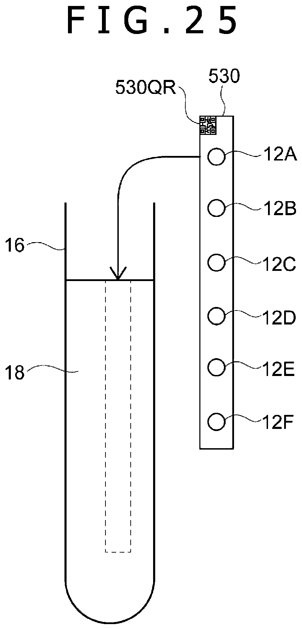

FIG. 25 is a view illustrating a state in which a reagent carrier 530 is input into the container 16 storing the urine 18 according to the first modification.

In the above-described embodiment, the reagent carrier 10 carries a plurality of images 14A to 14F (color charts) of the plurality of concentrations of each of the first component to the sixth component of the urine 18.

Meanwhile, in the first modification, the reagent carrier 530 does not carry these images 14A to 14F and is stored in the storage device 62 of the server 50. The reagent carrier 530 of the first modification carries six reagents 12A to 12F that react with each of a plurality of components of a body fluid of a living body and have a color development state changed according to the concentration of each component. The six reagents 12A to 12F stored in the storage device 62 of the server 50 are images of the respective reagents obtained by imaging the reagent carrier 530 in a predetermined imaging environment.

The user inputs the attribute data via the input device 21 of the smartphone 20. The user inputs the reagent carrier 530 into the container 16 in which the urine 18 is stored. The user images the reagent carrier 530 with the imaging device 28 within a predetermined period (for example, 10 seconds) from the input. The image data and attribute data of the reagent carrier 530 obtained by imaging the reagent carrier 530 by the imaging device 28 are transmitted to the server 50.

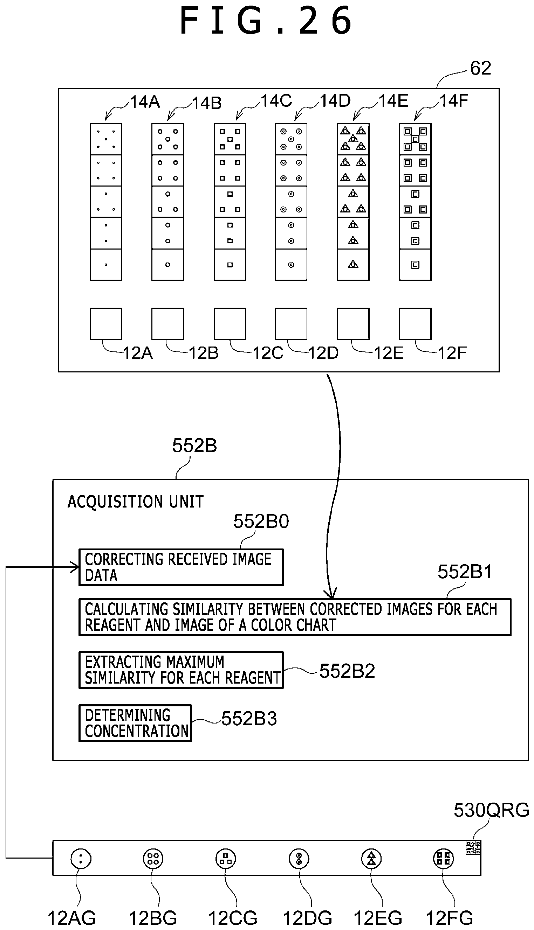

FIG. 26 is a diagram illustrating processing contents of an acquisition unit 52B according to the first modification. The acquisition unit 52B calculates an actual concentration of each component.

In Step 552B0, the acquisition unit 52B corrects the image data 12AG to 12CG of each reagent of the image data 530G of the reagent carrier 530 using the pre-trained model.

The pre-trained model is a pre-trained model pre-trained to correct a captured image of each reagent obtained by imaging the reagent carrier 530 in an imaging environment other than a predetermined imaging environment among a plurality of different imaging environments to a captured image of each reagent obtained by imaging the reagent carrier 530 in a predetermined imaging environment.

As a result, the image data 12AG to 12CG of each reagent of the image data 530G can be corrected to an image captured in the predetermined imaging environment.

In Step 552B1, the acquisition unit 52B calculates a similarity between the image corrected for each reagent and the image of the color chart. Specifically, for example, the acquisition unit 52B calculates the similarity between the corrected image of the reagent 12A and each of the plurality of images 14A.

In Step 552B2, the acquisition unit 52B extracts an image corresponding to the maximum similarity among the plurality of images 14A. In Step 552B3, the acquisition unit 52B determines the concentration from the image corresponding to the maximum similarity.

As described above, in the first modification, since the reagent carrier 530 does not carry the color chart, the reagent carrier 530 can be further downsized. In the first modification, the acquisition unit 52B corrects the image data 12AG to 12CG of each reagent of the image data 530G of the reagent carrier 530 using the pre-trained model. Therefore, the concentration of each component can be calculated more accurately.

### (Second Modification)

The user may visually confirm the image and the color chart after the color development of each reagent of the reagent carrier 10, input the concentration of each reagent via the input device 21, and transmit the concentration of each reagent from the smartphone 20 to the server 50, or the operator of the server 50 may input the concentration of each reagent via the input device 60 while viewing the image of the reagent carrier 10.

### (Third Modification)

The reagent of the reagent carrier 10 or 530 in each of the above examples develops a color with the concentration corresponding to the concentration of the component of urine when urine adheres thereto, but the technology of the present disclosure is not limited thereto, and the reagent may develop a color corresponding to the concentration of a component of urine when urine adheres thereto.

### (Fourth Modification)

The analysis device described in Japanese Patent No. 5542888 may be provided, and the concentration of each component of urine may be calculated from absorbance, reflectance, or transmittance by detecting transmitted light or scattered light in each image of the reagent of the reagent carriers 10 and 530.

### (Fifth Modification)

A concentration sensor for detecting the concentration of each component in urine described in JP H05-232107 A may be provided without using the reagent carrier, and the concentration of each component in urine may be calculated.

### (Other Modifications)

In the embodiment described above, the example in which the information processing program 62P is stored in the storage device 62 of the server 50 has been described, but the technology of the present disclosure is not limited thereto. For example, the information processing program 62P may be stored in a portable computer-readable non-transitory storage medium such as an SSD, a USB memory, or a magnetic tape. The information processing program 62P stored in the non-transitory storage medium is installed in the server 50. The processor 52 executes information processing according to the information processing program 62P.

Furthermore, the information processing program 62P may be stored in a storage device such as another computer or a server device connected to the server 50 via a network, and the information processing program 62P may be downloaded and installed in the server 50 in response to a request from the server 50.

Note that it is not necessary to store all of the information processing programs 62P in a storage device such as another computer or a server device connected to the server 50, and some of the information processing programs 62P may be stored.

In the above embodiment, the technology of the present disclosure has been described with a form example realized by a software configuration, but the technology of the present disclosure is not limited thereto, and a device including an application specific integrated circuit (ASIC), a field programmable gate array (FPGA), or a programmable logic device (PLD) may be applied. In addition, a combination of a hardware configuration and a software configuration may be used.

The following various processors can be used as hardware resources for executing the information processing described in the above embodiment. Examples of the processor include software, that is, a CPU that is a general-purpose processor functioning as a hardware resource that executes information processing by executing a program. In addition, examples of the processor include a dedicated electronic circuit which is a processor having a circuit configuration exclusively designed for executing specific processing such as FPGA, PLD, or ASIC. A memory is built in or connected to any processor, and any processor executes information processing by using the memory.

The hardware resource that executes the information processing may be configured by one of these various processors, or may be configured by a combination (for example, a combination of a plurality of FPGAs or a combination of a CPU and an FPGA) of two or more processors of the same or different types. The hardware resource that executes the information processing may be one processor.

As an example of the configuration including one processor, first, there is an aspect in which one processor is configured by a combination of one or more CPUs and software, and the processor functions as a hardware resource that executes information processing. Second, as represented by a system-on-a-chip (SoC) or the like, there is a form of using a processor that realizes a function of the entire system including a plurality of hardware resources for executing information processing by one integrated circuit (IC) chip. In this manner, the information processing is realized by using one or more of the above-described various processors as hardware resources.

Furthermore, more specifically, an electronic circuit in which circuit elements such as semiconductor elements are combined can be used as a hardware structure of these various processors. Furthermore, the above information processing is merely an example. Therefore, it is needless to say that unnecessary steps may be deleted, new steps may be added, or the processing order may be changed within a range not departing from the gist.

The contents described and illustrated above are detailed descriptions of parts according to the technology of the present disclosure, and are merely examples of the technology of the present disclosure. For example, the above description regarding the configuration, function, operation, and effect is a description regarding an example of the configuration, function, operation, and effect of the portion according to the technology of the present disclosure. Therefore, it is needless to say that unnecessary portions may be deleted, new elements may be added, or replacement may be made with respect to the above described and illustrated contents without departing from the gist of the technology of the present disclosure. Furthermore, in order to avoid complication and to facilitate understanding of the portion according to the technology of the present disclosure, in the description content and the illustrated content described above, description regarding technical common sense or the like that does not require any particular description in enabling implementation of the technology of the present disclosure is omitted.

All documents, patent applications, and technical standards described in this specification are incorporated herein by reference to the same extent as if each document, patent application, and technical standard were specifically and individually indicated to be incorporated by reference.

From the above disclosure, the following addendums are proposed.
1. An information processing device comprising:
   an acquisition unit that acquires a concentration of each of a plurality of components included in a body fluid of a living body; and
   a calculation unit that calculates a difference between the acquired concentration of each component of the body fluid of the living body and a target concentration of each component of a body fluid of a target living body determined by attribute data of the living body.
2. The information processing device according to addendum 1, wherein the acquisition unit acquires the concentration of each component of the body fluid of the living body by calculating the concentration of each component of the body fluid of the living body on a basis of a captured image obtained by imaging the plurality of reagents that has been changed by attachment of the body fluid to a reagent carrier that carries the plurality of reagents that react with each of the plurality of components of the body fluid of the living body and change a color development state (color/concentration) according to the concentration of each component.
3. The information processing device according to addendum 1 or 2, further comprising an output unit that outputs information related to the difference of each component.
4. The information processing device according to addendum 3, wherein the difference-related information for each component is at least one of
   information indicating an aspect of the difference of each component,
   information on the concentration of each component,
   a solution for eliminating a difference between a concentration of each component of the living body and the target concentration of each component of the body fluid of the target living body corresponding to the aspect of the difference of each component, and
   information on a store of a product for achieving the solution.
5. The information processing device according to addendum 4, wherein the solution is at least one of
   information on at least one of an ingested material recommended and a non-ingested material not recommended corresponding to the aspect of the difference of each component,
   recipe information of the ingested material, and
   advice information on a life aspect corresponding to the aspect of the difference of each component.
6. The information processing device according to addendum 5, wherein the information on the ingested material is at least one of a product name of the ingested material, information on a store where the ingested material is available, and an intake amount of the ingested material.
7. The information processing device according to addendum 5, wherein the advice information is generated using an artificial intelligence tool.
8. The information processing device according to addendum 2, wherein the acquisition unit calculates the concentration of each component of the body fluid of the living body on the basis of the captured images of the plurality of reagents that have changed due to attachment of the body fluid and an image of each of a plurality of concentrations of each of the plurality of components of a body fluid of a living body.
9. The information processing device according to addendum 8, wherein
   the image of each of the plurality of concentrations of each of a plurality of components of the body fluid of the living body is carried on the reagent carrier, and
   the acquisition unit calculates a concentration of each component of the body fluid of the living body based on the captured images of the plurality of reagents changed by attachment of the body fluid and the image of each of the plurality of concentrations of each of the plurality of components carried on the reagent carrier.
10. The information processing device according to addendum 8, further comprising
   a storage unit that stores the image of each of the plurality of concentrations of each of the plurality of components of the body fluid of the living body,
   wherein the acquisition unit calculates the concentration of each component of the body fluid of the living body based on the captured images of the plurality of reagents changed by attachment of the body fluid and the image stored in the storage unit.
11. The information processing device according to addendum 10, wherein
   the image of each of the plurality of concentrations of each of the plurality of components stored in the storage unit is created with a predetermined imaging environment as a reference, and
   the acquisition unit corrects the captured images of the plurality of reagents using a pre-trained model pre-trained to correct the captured images of the plurality of reagents obtained by imaging the reagent carrier in an imaging environment other than the predetermined imaging environment among a plurality of different imaging environments to the captured images of the plurality of reagents obtained by imaging the reagent carrier in the predetermined imaging environment, and calculates the concentration of each component of the living body on the basis of the corrected captured images of the plurality of reagents and data of an image stored in the storage unit.
12. The information processing device according to any one of addendums 8 to 11, wherein the acquisition unit compares, by image matching, the captured images of the plurality of reagents that have changed due to attachment of the body fluid with an image of each of a plurality of concentrations of each of a plurality of components of the body fluid of the living body, and calculates the concentration of each component of the living body from a result of the comparison.
13. The information processing device according to addendum 2 and any one of addendums 8 to 12, further comprising a receiving unit that receives the captured images of the plurality of reagents.
14. The information processing device according to any one of addendums 1 to 13, wherein the living body is an animal, and the body fluid is urine.
15. A program for causing a computer to function as the acquisition unit and the calculation unit according to any one of addendums 1 to 14.
16. A communication terminal comprising:
   a receiving unit that receives information related to the difference of each component according to addendum 3; and
   a display unit that displays information related to the difference of each component.
17. The communication terminal according to addendum 16, wherein the difference-related information for each component is at least one of
   information indicating an aspect of the difference of each component,
   information on the concentration of each component,
   a solution for eliminating a difference between a concentration of each component of the living body and a target concentration of each component of the body fluid of the target living body corresponding to the aspect of the difference of each component, and
   information on a store of a product for achieving the solution.
18. A communication terminal comprising:
   a receiving unit that receives information indicating an aspect of the difference of each component as the information related to the difference of each component according to addendum 3; and
   a display unit that displays information indicating the aspect of the difference of each component.
19. A payment amount calculation device comprising:
   a reading unit that reads information on a product attached to the product;
   a payment amount calculation unit that calculates, as a final payment amount, an amount obtained by subtracting an amount according to the information indicating an aspect of the difference displayed on the display unit according to addendum 18 from a payment amount determined in advance corresponding to the information of the product.
20. A point addition device comprising an addition unit that adds a point corresponding to the information indicating the aspect of the difference displayed on the display unit according to addendum 18 to a point acquired from a point carrier.
21. A communication terminal comprising:
   a receiving unit that receives information indicating the aspect of the difference of each component as the information related to the difference of each component according to addendum 3; and
   a transmission unit that transmits information indicating the aspect of the difference of each component.
22. A product providing device comprising:
   an acquisition unit that acquires (reads/receives) information indicating the aspect of the difference of each component from the communication terminal according to addendum 18 or 21; and
   a product providing unit that provides a product corresponding to the acquired information indicating the aspect of the difference.
23. A product providing device comprising:
   a manufacturing unit that manufactures a product;
   an acquisition unit that acquires (reads/receives) information indicating the aspect of the difference of each component from the communication terminal according to addendum 18 or 21; and
   a control unit that controls the manufacturing unit to manufacture a product corresponding to the acquired information indicating the aspect of the difference.
24. A communication terminal comprising:
   a receiving unit that receives information indicating an aspect of the difference of each component according to addendum 3 and data indicating a position of an ingested material determined according to the aspect of the difference in an area in which a plurality of ingested materials are arranged in a shop that sells the plurality of ingested materials determined corresponding to each of the plurality of aspects of the difference; and
   a presentation unit that presents a position of the ingested material in the area on the basis of data indicating the position of the ingested material determined according to the aspect of the difference in the area.
25. A communication terminal comprising:
   a receiving unit that receives information indicating the aspect of the difference of each component as the information related to the difference of each component according to addendum 3;
   an access unit that accesses a website that sells a plurality of types of ingested materials; and
   a display unit that displays the ingested material corresponding to the received information indicating the aspect of the difference among the plurality of types of ingested materials of the accessed website.
26. An information giving device that gives information indicating one of a plurality of aspects according to a difference between a concentration of each component of a body fluid of a living body and a target concentration of each component of a body fluid of a target living body determined by attribute data of the living body to a product or a packaging of the product.
27. A solution providing device comprising
   a storage unit that stores a solution for solving a difference between a concentration of each component of a body fluid of a living body and a target concentration of each component of a body fluid of a target living body, corresponding to each of a plurality of aspects according to a difference between the concentration of each component of the body fluid of the living body and the target concentration of each component of the body fluid of the target living body determined by attribute data of the living body; and
   a providing unit that provides the solution corresponding to a designated aspect.
28. A shelf that is partitioned according to each of a plurality of aspects according to a difference between a concentration of each component of a body fluid of a living body and a target concentration of each component of a body fluid of a target living body determined by attribute data of the living body, and stores an ingested material recommended to be ingested according to each aspect.
29. The shelf according to addendum 28, wherein
   the shelf is partitioned by a partition plate, and
   the partition plate is movably arranged on the shelf such that a size of a storage area defined by the partition plate is changed.
30. The shelf according to addendum 28 or 29, wherein the partitioned section is provided with a display unit that displays information indicating the aspect.
31. A movement amount calculation device for calculating a movement amount of the partition plate according to addendum 29, the movement amount calculation device comprising:
   a counting unit that counts the number of sales of the ingested material for each partitioned section; and
   a calculation unit that calculates a movement amount of the partition plate for making a size of a storage area of a partitioned section with a large number of sales larger than a size of a storage area of a partitioned section with a small number of sales according to the counted number of sales.

## Claims

1. An information processing device comprising:
an acquisition unit that acquires a concentration of each of a plurality of components included in a body fluid of a living body; and
a calculation unit that calculates a difference between the acquired concentration of each component of the body fluid of the living body and a target concentration of each component of a body fluid of a target living body determined by attribute data of the living body.

2. The information processing device according to claim 1, wherein the acquisition unit acquires the concentration of each component of the body fluid of the living body by calculating the concentration of each component of the body fluid of the living body on a basis of a captured image obtained by imaging the plurality of reagents that has been changed by attachment of the body fluid to a reagent carrier that carries the plurality of reagents that react with each of the plurality of components of the body fluid of the living body and change a color development state (color/concentration) according to the concentration of each component.

3. The information processing device according to claim 1, further comprising an output unit that outputs information related to the difference of each component.

4. The information processing device according to claim 3, wherein the difference-related information for each component is at least one of
information indicating an aspect of the difference of each component,
information on the concentration of each component,
a solution for eliminating a difference between a concentration of each component of the living body and the target concentration of each component of the body fluid of the target living body corresponding to the aspect of the difference of each component, and
information on a store of a product for achieving the solution.

5. The information processing device according to claim 4, wherein the solution is at least one of
information on at least one of an ingested material recommended and a non-ingested material not recommended corresponding to the aspect of the difference of each component,
recipe information of the ingested material, and
advice information on a life aspect corresponding to the aspect of the difference of each component.

6. The information processing device according to claim 5, wherein the information on the ingested material is at least one of a product name of the ingested material, information on a store where the ingested material is available, and an intake amount of the ingested material.

7. The information processing device according to claim 5, wherein the advice information is generated using an artificial intelligence tool.

8. The information processing device according to claim 2, wherein the acquisition unit calculates the concentration of each component of the body fluid of the living body on the basis of the captured images of the plurality of reagents that have changed due to attachment of the body fluid and an image of each of a plurality of concentrations of each of the plurality of components of a body fluid of a living body.

9. The information processing device according to claim 8, wherein
the image of each of the plurality of concentrations of each of a plurality of components of the body fluid of the living body is carried on the reagent carrier, and
the acquisition unit calculates a concentration of each component of the body fluid of the living body based on the captured images of the plurality of reagents changed by attachment of the body fluid and the image of each of the plurality of concentrations of each of the plurality of components carried on the reagent carrier.

10. The information processing device according to claim 8, further comprising
a storage unit that stores the image of each of the plurality of concentrations of each of the plurality of components of the body fluid of the living body,
wherein the acquisition unit calculates the concentration of each component of the body fluid of the living body based on the captured images of the plurality of reagents changed by attachment of the body fluid and the image stored in the storage unit.

11. The information processing device according to claim 10, wherein
the image of each of the plurality of concentrations of each of the plurality of components stored in the storage unit is created with a predetermined imaging environment as a reference, and
the acquisition unit corrects the captured images of the plurality of reagents using a pre-trained model pre-trained to correct the captured images of the plurality of reagents obtained by imaging the reagent carrier in an imaging environment other than the predetermined imaging environment among a plurality of different imaging environments to the captured images of the plurality of reagents obtained by imaging the reagent carrier in the predetermined imaging environment, and calculates the concentration of each component of the living body on the basis of the corrected captured images of the plurality of reagents and data of an image stored in the storage unit.

12. The information processing device according to claim 8, wherein the acquisition unit compares, by image matching, the captured images of the plurality of reagents that have changed due to attachment of the body fluid with an image of each of a plurality of concentrations of each of a plurality of components of the body fluid of the living body, and calculates the concentration of each component of the living body from a result of the comparison.

13. The information processing device according to claim 2, further comprising a receiving unit that receives the captured images of the plurality of reagents.

14. The information processing device according to claim 1, wherein the living body is an animal, and the body fluid is urine.

15. A program for causing a computer to function as the acquisition unit and the calculation unit according to claim 1.
